# EUROPEAN PATENT APPLICATION

(11) **EP 2 210 616 A1**
(43) Date of publication of application: **28.07.2010**
(21) Application number: 09305055.7
(22) Date of filing: 21.01.2009
(51) Int. Cl.: A61K 47/48, A61P 35/00

(54) **Multifunctional stealth nanoparticles for biomedical use**

(71) Applicant: Centre National de la Recherche Scientifique, 75016 Paris Cedex 16 (FR); Université de Strasbourg, 67000 Strasbourg (FR); Institute of Macromolecular Chemistry AS CR v.v.l., 162 06 Praha 6 (CZ)
(72) Inventor: Schneider, Grégory F., 67380, LINGOLSHEIM (FR); Decher, Gero, 77694, KEHL-MARLEN (DE); Ulbrich, Karel, PRAGUE (CZ); Subr, Vladimir, 276 01, MELNIK (FR)
(74) Representative: Warcoin, Jacques

(57) **Abstract**

The present invention relates to the field of drug delivery nanosystems. More precisely, the present invention concerns a copolymer with advantageous properties for the outer coating of various nanoparticles. Said copolymer comprises at least three types of monomers with stealthy, coupling and therapeutic properties respectively, as well as an optional fourth type of monomers with targeting properties. The present invention also relates to core-shell or hollow shell nanoparticles coated by an external layer of the copolymer according to the invention. Several types of core-shell nanoparticles are envisaged. The invention also concerns methods for preparing said nanoparticles, as well as pharmaceutical compositions or medicaments comprising them.

## Description

### FIELD OF THE INVENTION

The present invention relates to the field of drug delivery nanosystems. More precisely, the present invention concerns a copolymer with advantageous properties for the outer coating of various nanoparticles. Said copolymer comprises at least three types of monomers with stealthy, coupling and therapeutic properties respectively, as well as an optional fourth type of monomers with targeting properties. The present invention also relates to core-shell or hollow shell nanoparticles coated by an external layer of the copolymer according to the invention. Several types of core-shell nanoparticles are envisaged. The invention also concerns methods for preparing said nanoparticles, as well as pharmaceutical compositions or medicaments comprising them, and their use as tumor-killing heat inducers for magnetic and electromagnetic hyperthermia treatments thanks to the core/shell architecture of the composition.

### BACKGROUND ART

Cancer is the second mortality cause in developed countries, with more than eleven millions people diagnosed as suffering from cancer each year, and seven millions people killed by cancer each year in the world according to World Health Organization (WHO). Cancer is thus an important public health problem in developed countries, and the ageing of their population will cause these numbers to continue to increase even if age-specific rates remain constant.

Conventional treatments against cancer include surgery, radiotherapy and chemotherapy. While surgery usually does not have many deleterious effects, it is not always possible and it is also usually not sufficient to cure cancer, since tumor cells may have escaped surgical removal. Thus, radiotherapy and chemotherapy are necessary, even when surgery is possible.

However, while most drugs may have deleterious effects, anticancer drugs are among those resulting in the worse adverse effects. Indeed, anticancer drugs are usually cytotoxic active agents with some preference for tumor cells, but which also display toxicity on other cells due to insufficient specificity for tumor cells, thus resulting in often serious adverse reactions. Although radiotherapy is more localized, it is also not specific of tumor cells and thus also results in serious adverse effects on healthy surrounding cells.

There is thus a very important need for alternative cancer treatments with increased specificity for tumor cells and thus decreased adverse effects.

Recently, it was proposed to use nanosized systems for the vectorization of anticancer drugs. Indeed, it has been found that nanoparticles preferentially accumulate into tumor tissues. This phenomenon, which is known as EPR effect (Enhanced Permeability and Retention effect), is linked to an enhanced permeability of the endothelium of the vessels of the tumour tissue and a missing or limited lymphatic drainage. The tumor tissue thus constitutes a quite efficient retention filter for nanoparticles. Said EPR effect appears to be limited to nanoparticles with a diameter of about 10 to about 150 nm.

As a result, many nanosized systems have been developed in recent years in order to improve delivery of anticancer drugs.

However, therapeutic, and also diagnostic, applications of most described nanosystems are still seriously limited by several factors such as the potential aggregation of the nanoparticles in physiological media or their short circulation time *in vivo* due to elimination from the blood stream by macrophages of the mononuclear phagocytic system (MPS) or significant uptake by the liver before reaching any target.

Other important drawbacks for potential applications include a low loading capacity, a lack of site-specific targeting or limited release of the carried biologically active compound at the site of interest.

In order to obtain a truly efficient nanosystem for delivering anticancer drugs to tumor tissues, a nanosystem should fulfil the following criteria:
1) a significant bioavailability of the anticancer drug to cancer cells, thus implying:
   a) a well adjustable size in combination with a small polydispersity of the nanoparticle carrier in order to benefit from the EPR effects.
   b) a high stability in physiological media in order to prevent aggregation, which would result both in loss of the EPR effect and accumulation in liver
   c) "stealthiness" and thus protection against uptake by macrophages
   d) A sufficient drug-loading capacity in order to reach sufficient drug concentration in cancer cells
   e) An efficient drug release in tumor cells.
   f) An efficient targeting of tumor and cancer cells (active and passive targeting).
   g) The capacity to reach tumor cells either by passive targeting, thanks to a size taking advantage of the EPR effect, and/or by active targeting thanks to the presence of a targeting ligand in the nanosystem.
2) a low toxicity to non-tumor cells, thus implying:
   a) a low toxicity of the carrier system in case of prolonged use,
   b) a low or inexistent drug release into blood circulation, before reaching cancer cells

Nevertheless, it appears that while some existing nanosystems may fulfil one or more of these criteria, none of the existing systems permit to fulfil all of these criteria.

Patent US 7,101,575 describes core/shell nanoparticles made of a core nanoparticle onto which multiple layers of polyelectrolytes are deposited using Layer By Layer (LBL) technology, the interactions between successive layers of polyelectrolytes being preferably electrostatic, although hydrogen bonds or hydrophobic binding may be used. Successive layers are thus interacting with each other by weak binding. No covalent binding between layers is described.

This document also describes that the core particle may be dissolved in order to obtain hollow shell nanoparticles. Various agents (including therapeutic agents) may then be included into the hollow shell. It is also indicated that permeability of the shell is adapted so that the agent be trapped into the shell, thus being protected from potentially harmful components present in blood circulation. However, the mechanism by which the therapeutic agent is released in cancer cells is not clear, and the release is not specifically selective in cancer cells.

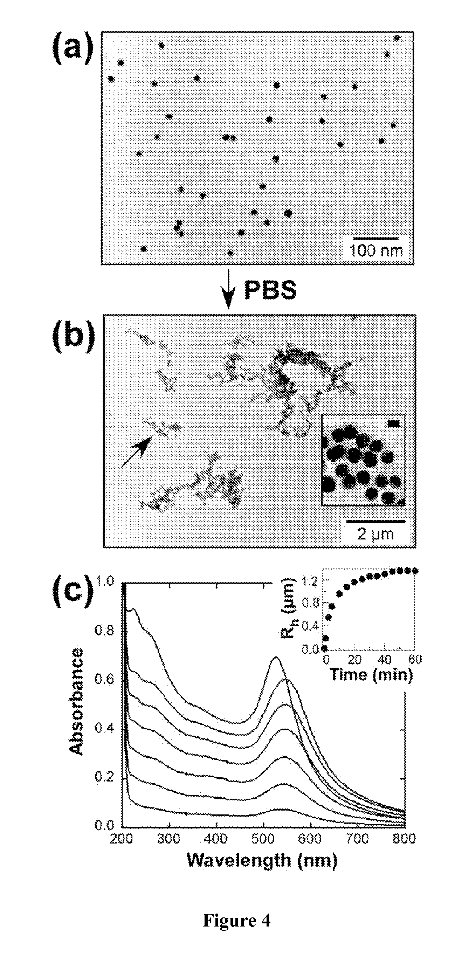

In view of what is described in this document, it thus appears that while the proposed nanosystem fulfils criteria 1) a), maybe 1) d), and 2 a) and b), it does not fulfil other criteria. In particular, while such a system may have high stability in pure water, aggregation of the micro or nanoparticles is observed in physiological media (see examples of the present application, especially Figure 4). In addition, stealthiness needs the presence on the outer surface of a highly hydrated, non charged component, while in this document the outer polyelectrolyte layer is a charged polymer, thus resulting in an absence of stealthiness and thus opsonization by macrophages. In addition, as mentioned above, it is not clear how the trapped drug may be efficiently released in cancer cells.

Other nanosystems have addressed the problem of stealthiness and aggregation.

For instance, application US2002/0192814 describes colloid metal core nanoparticles onto which is adsorbed an active agent, polyethylene glycol (PEG) being then adsorbed on all sites not bearing the active agent. PEG molecules confer stealthiness to the PEG-derivative nanoparticles, which are shown to accumulate selectively into tumors while organs of the MPS (liver, spleen, ...) remain clean.

While the nanosystem described in this application appears to fulfil criteria 1)b), 1)c), 1)e) as defined above, it does not seem to fulfil other criteria. In particular, the size of the obtained nanoparticle may not be easily modulated in order to obtain a particular desired dimension, since only the size of the core particle may be modulated. As a result, depending on the application and the desired global dimension, it may be necessary to change the core particle. In addition, the drug-loading capacity of such a system is limited since the active agent may be adsorbed on only one surface (e.g. the surface of the colloidal core). Furthermore, since the active agent is only adsorbed onto the core particle, its release may start in blood circulation, before reaching tumor cells, thus resulting both in potential toxicity and decreased drug release in tumor cells and thus decreased toxicity.

Nanospheres made of a self assembled complex of stealthy polymer in which an active agent is dispersed have also been proposed. However, although such systems appear to avoid aggregation and opsonisation by macrophages, the amount of active agent that may be dispersed into the nanosphere cannot be well adjusted and since this active agent is only dispersed into the nanosphere, its release may start in blood circulation, before reaching tumor cells, thus resulting both in potential toxicity and decreased drug release in tumor cells and thus decreased toxicity.

Rather than generating nanoparticles, other research groups have explored the possibility to use stealthy polymers covalently linked to active agents for the delivery of these active agents to target cells. A particularly well described such stealthy polymer is a *N-*(2-hydroxypropyl)methacrylamide (HPMA) copolymer carrying oligopeptide side chains with bound to doxorubicin. HPMA provides a hydrophilic backbone that limits phagocytosis and uptake by the liver. In addition, while doxorubicin is covalently linked to HPMA, it is not active and the active agent can only be released by lysosomal enzymes once the polymer has entered cells.

While this type of polymer may be useful, it does not have a well controlled and adjustable form and size, and it is thus not optimal for benefiting of the EPR effect.

In addition, such a polymer with a highly hydrated non charged backbone is not suitable for use in nanoparticles described in above cited patent US 7,101,575 using electrostatic, hydrogen bond, or hydrophobic interactions. In a similar way, if such a polymer was used instead of PEG to be adsorbed onto colloid metal particles with an active agent already adsorbed onto it as described in application US2002/0192814, the global size of the obtained nanoparticle would still not be adjustable.

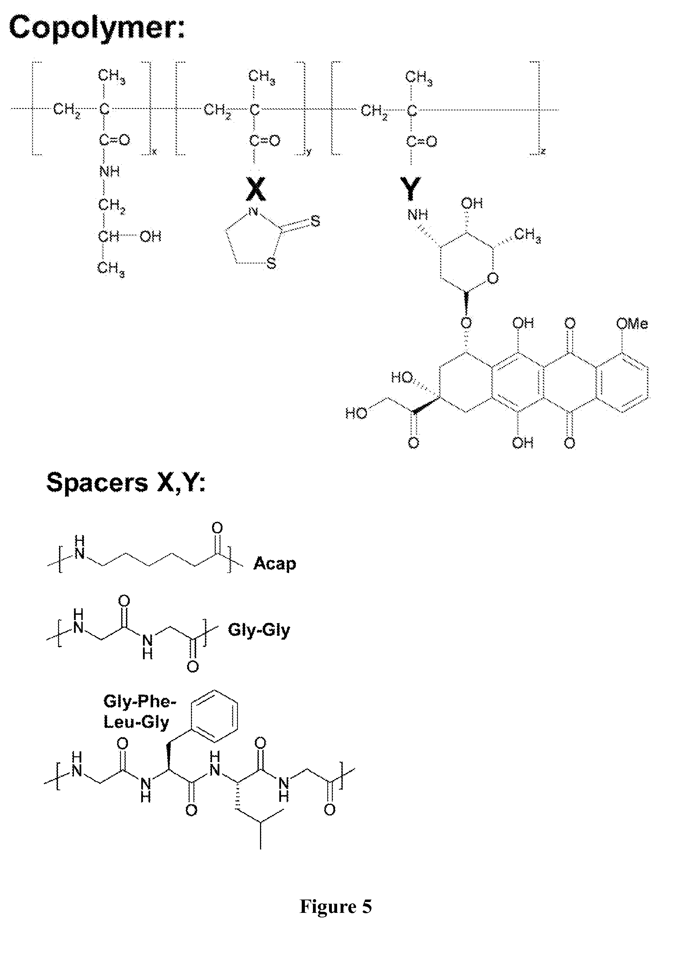

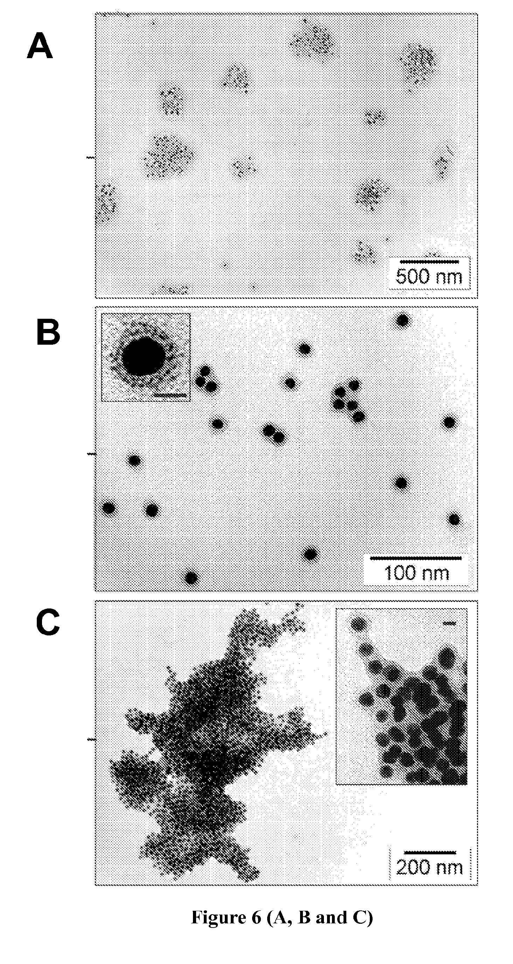

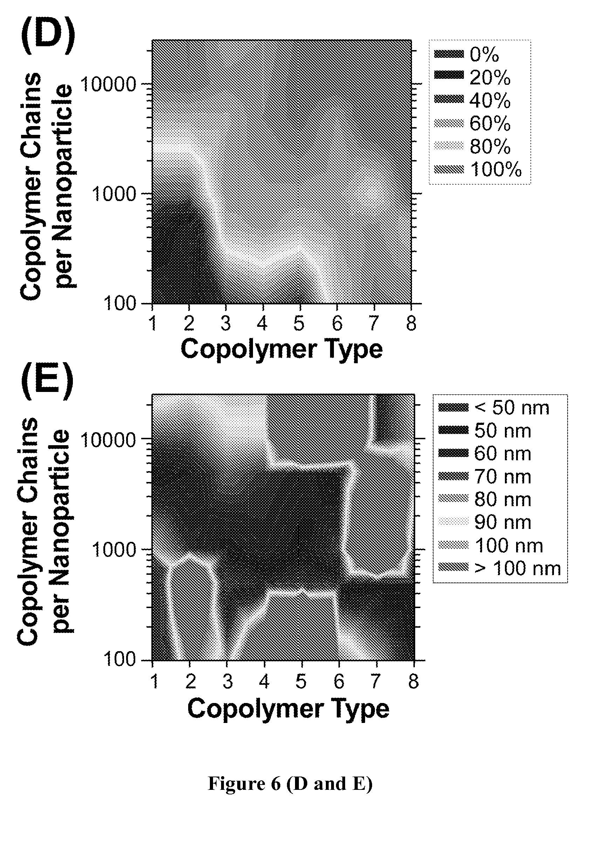

Application US 2006/0275250 describes (see notably Figures 5 and 6) *N*-(2-hydroxypropyl)methacrylamide (HPMA) derived copolymers comprising reactive thiazoline-2-thione groups, either on side chains of particular modified monomers or at the end of the polymer chain. Among *N-*(2-hydroxypropyl)methacrylamide (HPMA) derived copolymers comprising reactive thiazoline-2-thione groups at the end of the polymer chain, are described polymers that also comprise modified monomers linked through an oligopeptide spacer molecule to a doxorubicin molecule, and optionally modified monomers linked through an oligopeptide spacer molecule to an antibody.

This type of polymer may be used alone, in which case it also has the drawbacks of the simpler above described HPMA copolymer carrying oligopeptide spaced doxorubicin side-groups, i.e. it does not have a well controlled and adjustable form and size, and it is thus not optimal for benefiting of the EPR effect.

Application US 2006/0275250 also describes that *N*-(2-hydroxypropyl)methacrylamide (HPMA) derived copolymers comprising reactive thiazoline-2-thione groups on the side chain or at the end of the polymer chain, but with no grafted therapeutic agent, may be used to modify gene delivery systems such as polymer complexes of DNA or plasmids, or adenoviruses. In this case, the therapeutic agent is a gene encoded by the gene delivery system and the HPMA derived copolymer comprising reactive thiazoline-2-thione groups is only used for its stealthiness. In any case, such a HPMA derived copolymer comprising reactive thiazoline-2-thione groups cannot be used in nanoparticles described in above cited patent US 7,101,575 using electrostatic, hydrogen bond, or hydrophobic interactions.

There is thus a need for alternate nanoobjects and nanosystems (e.g., a system, or object, with a size ranged from one to one thousand nanometers) for delivering anticancer drugs to cancer cells with increased efficiency and decreased toxicity.

The inventors have developed a new stealthy copolymer based on the previously described *N-*(2-hydroxypropyl)methacrylamide (HPMA) copolymer carrying an oligopeptide spaced therapeutic agent, which comprises an additional third type of monomers with thiazoline-2-thione (TT) groups which permit to add a layer of this copolymer to a core/shell nanoparticle made of a core nanoparticle and of successive layers of polyelectrolytes as described in patent US 7,101,575. Due to the presence of the additional third type of monomers, covalent linkage to nanoparticles prepared by LBL technology may be used, so that the stealthy copolymer is covalently linked to the preceding charged polyelectrolyte layer.

While application US 2006/0275250 describes HPMA derived copolymers comprising one reactive thiazoline-2-thione group at the end of the polymer chain that also comprise modified monomers linked through an oligopeptide spacer molecule to a doxorubicin molecule, the copolymer developed by the inventors is different since it comprises several TT groups on the side chains of a proportion of monomers, and not only one at the end of the polymer chain. This is very important, since polymers with only one TT group at the end of the polymer chain described in US 2006/0275250 are not adapted for covalent binding to the preceding charged polyelectrolyte layer using covalent LBL technology, because only one TT group at the end of the polymer chain will result in a very low reactivity in the covalent LBL reaction and thus in a very low efficiency. In addition, these polymers are used for grafting an antibody at the end of the polymer before direct in vivo administration, and not for coating nanoparticles.

Moreover, although US 2006/0275250 also describes HPMA derived copolymers comprising reactive thiazoline-2-thione groups on the polymer side chain, these polymers do not comprise any "grafted" therapeutic agent, i.e. the therapeutic agent is not incorporated (linked to the polymeric backbone through a cleavable spacer) by co-monomers containing it (as opposed to grafting the therapeutic agent after the polymerization).

More importantly, US 2006/0275250 does not provide any motivation for a skilled artisan to prepare the polymer developed here by the inventors. Indeed, HPMA derived copolymers comprising one reactive thiazoline-2-thione group at the end of the polymer chain are sufficient for attaching the polymer to most carrier comprising reactive amine groups, they are not really adapted for depositing an outer layer onto LBL nanoparticles using covalent LBL technology because their reactivity with amine groups of the preceding layer would be very low, in particular if the polymerization degree is over 50-100.

Moreover, HPMA derived copolymers comprising reactive thiazoline-2-thione groups on the polymer side chain without grafted therapeutic agent are suggested to be used for:
- preparing HPMA derived copolymers in which a therapeutic agent, and optionally an antibody, replace the TT groups. Even when both a therapeutic agent and an antibody are linked to the polymer, the reaction is a one step reaction starting from HPMA derived copolymers comprising reactive thiazoline-2-thione groups on the polymer side chain, which is much simpler than preparing the copolymer developed by the inventors and only then grafting the antibody instead of the TT groups.
- Conferring stealthiness to gene delivery systems expressing a gene of interest. In this case, the therapeutic agent is already expressed by the gene delivery system and there is thus no reason to add monomers linked through a spacer molecule to a therapeutic agent.

More importantly, prior developing the copolymer described in the present invention, the inventors specifically attempted to use the HPMA derived copolymers comprising reactive thiazoline-2-thione that were described in US 2006/0275250. The only possible structure described in US 2006/0275250 that can fulfil i) nanoparticle coupling and ii) selective drug release is a copolymer made out of two monomers, the first being HPMA and the second being *N*-Methacryloylglycyl-DL-phenylalanylleucylglycine-thiazoline-2-thione (where a partial percentage of TT groups were aminolyzed with doxorubicin after copolymerization). Such a copolymer yielded aggregated nanoparticles and a functionalization yield below 10%. The inventors therefore have found that it was necessary to use a copolymer bearing at least three different monomeric units (e.g. stealthy, coupling, drug release; and more preferably a fourth targeting comonomer) in order to obtain non aggregated nanoparticles.

The copolymer according to the present invention has thus been developed by the inventors for a very specific application with particular constraints, in order to significantly improve the nanoparticles described in patent US 7,101,575 by adding a layer of this copolymer, covalently linked to the preceding charged polyelectrolyte layer.

Indeed, the resulting nanoparticles according to the invention are very advantageous, since they fulfil all desirable criteria cited above, i.e.:
1) a significant bioavailability of the anticancer drug to cancer cells, because of:
   a) their well adjustable size thanks to the selection of a particular number of intermediate charged polyelectrolyte layers, in combination with a small polydispersity of the nanoparticle. This permits to efficiently benefit from the EPR effect.
   b) a high stability in physiological media in order to prevent aggregation. This property is conferred by the uncharged outer layer of HPMA derived polymer.
   c) Their stealthiness conferred by the outer layer of HPMA derived stealthy polymer. This permits to avoid opsonisation by macrophages.
   d) A sufficient drug-loading capacity thanks to the increased area of the outer layer compared to adsorption directly onto the core particle. In addition, as described below, several layers of the copolymer, separated by polyamine layers to which they are covalently linked, may be added to the nanoparticle as described in patent US 7,101,575, thus permitting to further increase and adjust the drug-loading capacity. This property permits to reach sufficient drug concentration in cancer cells
   e) An efficient drug release in tumor cells, thanks to the presence of the lysosomal specific cleavage of the spacer between the polymer and the therapeutic agent. This permits to be sure that the therapeutic agent is actually released once it has reached its target.
2) a low toxicity to non-tumor cells, because of:
   c) a low toxicity of the carrier system in case of prolonged use. In particular, gold core nanoparticles are known not to display acute toxicity. In addition, it is possible to dissolve the core particle in order to decrease the risk of long-term chronic toxicity.
   d) a low or inexistent drug release into blood circulation, before reaching cancer cells. This is possible thanks to the covalent attachment of the therapeutic agent to the polymer backbone via a spacer that is only cleaved in lysosomes.

### DESCRIPTION OF THE INVENTION

The present invention thus relates to a linear copolymer comprising monomer units of following formulas (I), (II), (III) and (IV): 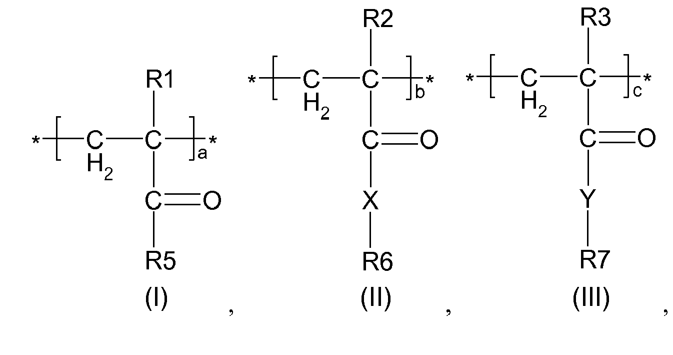 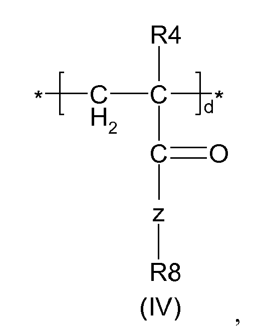 and
wherein
R1, R2, R3 and R4 are each independently selected from H and CH₃;
X, Y, and Z are spacer molecules independently selected from the group consisting of a peptide of 1-20 amino acids, an oligonucleotide of 1-20 nucleotides, a C₁-C₂₀ alkyl, and -(CH₂CH₂O)ᵢ wherein i is an integer in the range of 1-20;
R5 represents a stealthy molecule selected from the group consisting of:
-NHR9 wherein R9 is a hydroxylated C₁-C₆ alkyl, advantageously -CH₂-CHOH-CH₃; 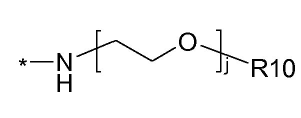 wherein R10 is selected from the group consisting of a hydrogen atom and a hydroxylated C₁-C₆ alkyl; and 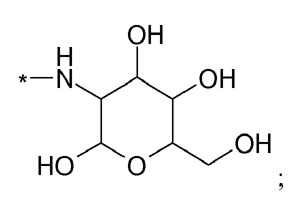 R6 represents a coupling molecule selected from the group consisting of:
   -SH, -NH₂ (including BOC and FMOC amines; e.g. respectively t-butoxycarbonyl- and 9-fluorenylmethoxycarbonyl amine), -COOH, -SO₃Na, -S-S-R11, -O-PO₃²⁻, polyphosphate, -(-(PO₄⁻)ₙ-R11,
      -N⁺(CH₃)₂-R11, -O-Si(OR11)₃, 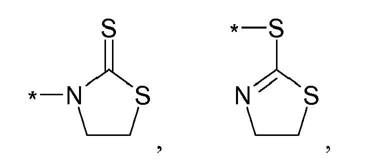 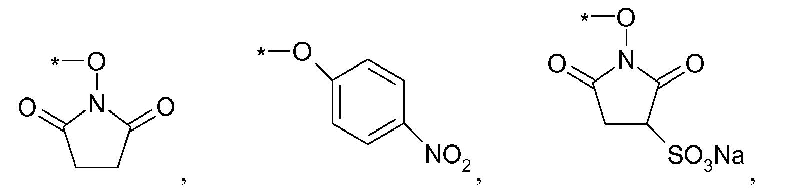 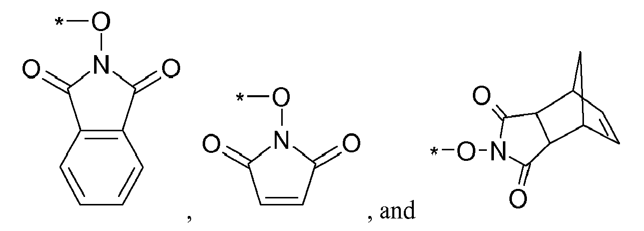 wherein R11 is selected from the group consisting of a hydrogen atom, a
      C₁-C₁₀ alkyl and a hydroxylated C₁-C₆ alkyl;
      R7 is a therapeutic agent;
      R8 is a targeting molecule; and
      a, b, c and d correspond to the percentage of each monomer in the copolymer and:
      a is in the range of 50-90%, preferably 80-90%,
      b is in the range of 4-49%, preferably 4-19%,
      c is in the range of 1-40%, preferably 1-10%,
      d is in the range of 0-40%, preferably 0-9%, and
         preferably a + b + c + d = 100%,
         and * indicates the position involved in binding with another group.

Advantageously, the copolymer according to the present invention is especially designed to be coupled with a nano-object (size range of the nanoobject: 1-1000 nm)

For the purpose of the present invention, the term "(C₁-Cₙ)alkyl", wherein n is a positive integer, is intended to mean any linear or branched saturated hydrocarbon radical having from one to n carbon atoms. For instance, a "(C₁-C₆)alkyl" is intended to mean any linear or branched saturated hydrocarbon radical having from one to six carbon atoms. Examples of (C₁-C₆)alkyl groups include a methyl, an ethyl, a propyl or a tert-butyl group.

For the purpose of the present invention, the term "hydroxylated (C₁-Cₙ)alkyl",
wherein n is a positive integer, is intended to mean a (C₁-Cₙ)alkyl as defined above in which at least one hydrogen atom has been substituted by a hydroxyl (OH) group. Said hydroxyl group may be at the end of the hydrocarbon chain, in which case said hydroxylated (C₁-Cₙ)alkyl may be referred to as an OH-terminated (C₁-Cₙ)alkyl. Alternatively, the OH substituent may be situated at a non terminal position in the (C₁-Cₙ)alkyl. Examples of hydroxylated (C₁-C₆)alkyls include -CH₂-CH₂-OH, -CH₂-CH₂-CH₂-OH (OH-terminated) and -(CH₂)-CHOH-CH₃.

For the purpose of the present invention, the term "peptide" is intended to mean a short polymer formed from the linking through an amide (or peptide) bond, in a defined order, of α-amino acids, which are defined by the following formula: 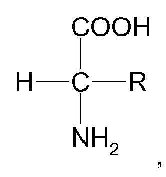 wherein R represents the side chain of the amino acid.

Naturally occurring amino acids may be used, to which it is referred using abbreviations displayed in following Table 1:

**Table 1. Naturally occurring amino acids and their 3-letters and 1-letter abbreviations**

| **Amino Acid** | **3-Letters** | **1-Letter** |
|---|---|---|
| Alanine | Ala | A |
| Arginine | Arg | R |
| Asparagine | Asn | N |
| Aspartic acid | Asp | D |
| Cysteine | Cys | C |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

Alternatively, non naturally occurring amino acids with the above described formula may also be used.

For the purpose of the present invention, the term "oligonucleotide" is intended to mean a short polymer formed from the linking in a defined order, of nucleotides.

A nucleotide is composed of a ring of nitrogen, carbon and oxygen atoms, a five carbon sugar (together referred to as a nucleoside) and one phosphate group. The most common nucleotides can be divided into two groups (purines: adenine and guanine; and pyrimidines: cytosine, thymine and uracil) based on the structure of the nitrogenous base.

The joined five carbon sugar (or pentose) may be either ribose or deoxyribose (in this case, they are usually called deoxynucleotides).

Common nucleosides (junction of base and pentose) thus include adenosine, guanosine, cytidine, uridine, which are used in RNA, and deoxyadenosine, deoxyguanosine, deoxycytidine, deoxythymidine, which are used in DNA.

However, oligonucleotides according to the invention may be modified by using modified bases, or a modified backbone between nucleotides.

In a preferred embodiment, in the copolymer according to the invention, R1, R2, R3 and R4 are each independently selected from H and CH₃.

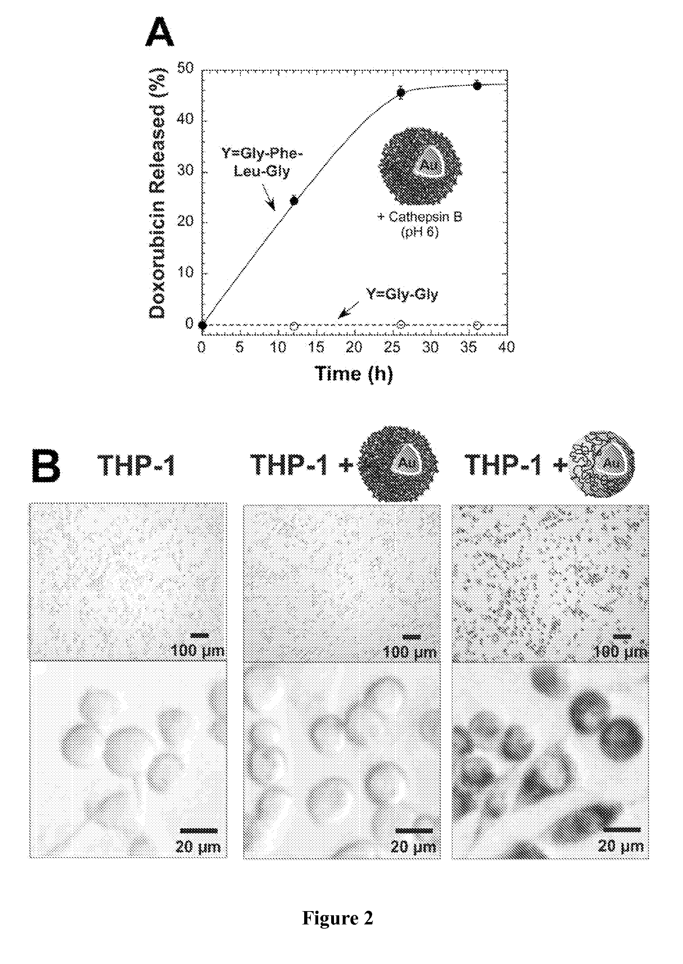

In the copolymer according to the invention, X, Y, and Z are spacer molecules included in monomer units of formula (II), (III) and (IV) respectively, which are independently selected from the group consisting of a peptide of 1-20 amino acids, an oligonucleotide of 1-20 nucleotides, a C₁-C₂₀ alkyl, and -(CH₂CH₂O)ᵢ wherein i is an integer in the range of 1-20. In a preferred embodiment, X, Y, and Z are selected from peptide of 1-20 amino acids. Some of these 1-20 amino acid peptides are recognized and cleaved by restriction enzymes, while others are not. Examples of 1-20 amino acid peptides that are recognized and cleaved by restriction enzymes include peptides of 2-20 amino acids comprising an amino acid sequence selected from the group consisting of FL, LF, FF, FK, VC, GFA, LALA (SEQ ID NO:1), GFLG (SEQ ID NO:2), GLFG (SEQ ID NO:3), GFFG (SEQ ID NO:4), GFYA (SEQ ID NO:5), GFAL (SEQ ID NO:6), GFLFG (SEQ ID NO:7), and YGGFL (SEQ ID NO:8). Examples of peptides that are not recognized and cleaved by restriction enzymes of lysosomes include peptides of amino acid sequence GG (Figure 2A) or (P)ₙ (where P is selected from the list of 20-naturally occurring amino acid, and n is a positive integer ranged from 1 to 10, or any of the non degradable spacers X or Z mentioned above, including non degradable oligopeptide of oligonucleotide sequences.

The copolymers of the present invention can only be composed of monomer units that are not reacting with each other during copolymerization. Therefore, reactive groups such as the coupling groups R6, therapeutic agents R7, and targeting moieties R8, must fulfil this later criteria of not inter-reacting under the polymerization conditions presented here (e.g. solvent, DMSO; temperature, 60 °C; copolymerization time of at least six hours). For that purpose, a skilled artisan can use the strategy of chemical protection/deprotection of potentially reactive amino acids (especially, but not limited to lysines and cysteines) present in the spacers X, Y or Z, more preferably by protecting primary amines and sulphur residues with so called protecting groups (strategies for chemical protection are well known, see the reviews from Orain and Schelhaas for more details: Orain, D.; Ellard, J.; Bradley, M. *Journal of combinatorial Chemistry* **2002**, *4*, 1-16; Schelhaas, M.; Waldmann, H. *Angewandte Chemie-International Edition* **1996**, *35*, 2056-2083).

As can be seen from above, the copolymer according to the invention comprises four types of monomer units with various representation percentages a, b, c and d. Although the copolymer according to the invention preferably does not contain other types of monomer units, in which case a + b + c + d = 100%, the presence of a small percentage of other monomer units is possible. Such additional monomers may for instance result from side products from preparation of the monomers.

The four types of monomer units have distinct intended functions, as described below.

Monomer units of formula (I) are used to confer stealthiness to the copolymer, thus permitting it to escape macrophages uptake, and also preventing aggregation of copolymer coated nanosystems. Stealthiness is conferred by the stealthy molecule R5 present in the side chain of monomer units of formula (I).

A "stealthy molecule" is intented to mean any molecule that is able to prevent macrophage uptake and aggregation. For obtaining this effect, such a molecule should be uncharged and hydrophilic, as are all above cited R5 molecules.

In the copolymer according to the invention, R5 is preferably selected from the group consisting of -NH-CH₂-CHOH-CH₃, -NH-CH₂-CH₂-OH, -NH-CH₂-CH₂-CH₂-OH, -NH-(CH₂)₂-O-(CH₂)₂-OH, 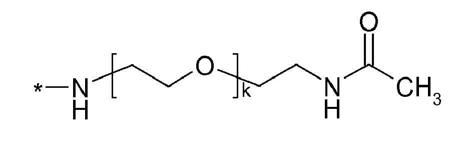 wherein k is between 1 and 20, 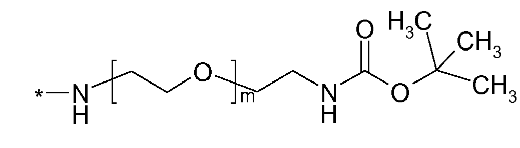 wherein m is between 1 and 25, and

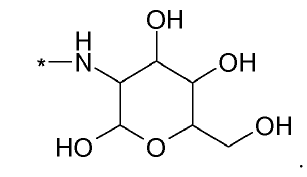

More preferably, R5 is selected from the group consisting of -NH-CH₂-CHOH-CH₃, -NH-CH₂-CH₂OH, -NH-CH₂-CH₂-CH₂OH, -NH-(CH₂)₂-O-(CH₂)₂-OH. Most preferably, R5 is -NH-CH₂-CHOH-CH₃ or -NH-CH₂-CH₂-CH₂OH.

As explained above, R1 is preferably selected from H and CH₃. More preferably, R1 is CH₃.

Particularly preferred monomer units of formula (I) include: 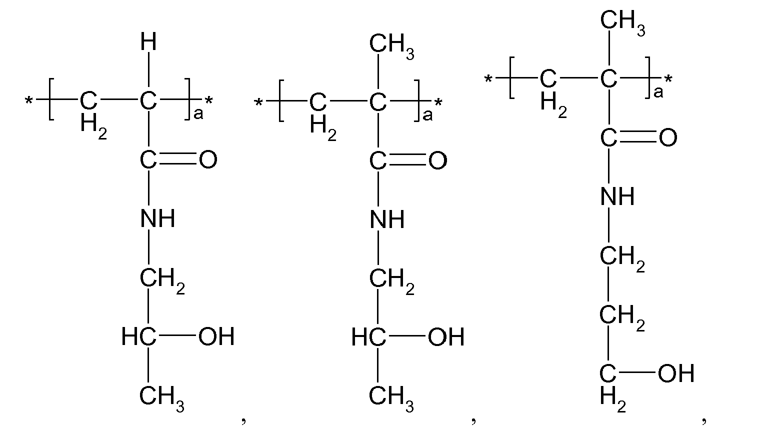 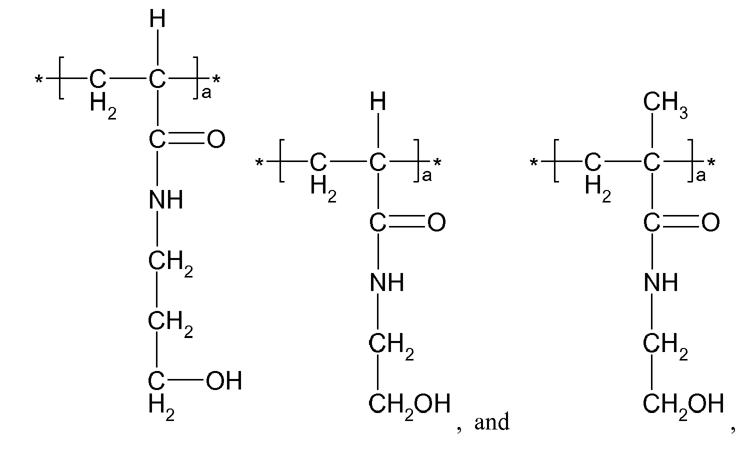 wherein a is as defined above.

A specifically preferred monomer unit of formula (I) is N-(2-Hydroxypropyl)methacrylamide (HPMA) of formula: 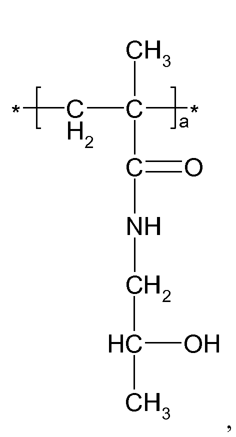 wherein a is as defined above.

In addition, for the resulting copolymer/nanoobject conjugate to be stealthy, it is necessary that at least 50%, and preferably more than 50%, i.e. at least 55%, at least 60%, at least 65%, more preferably at least 70%, at least 75%, and most preferably at least 80% of all monomer units are of formula (I). However, the percentage a of monomer units of formula (I) should not go beyond about 90%, so that other monomer units may have a sufficient percentage. Thus, the percentage a of monomer units of formula (I) is in the range of 50-90%, preferably 55-90%, 60-90%, 65-90%, more preferably 70-90%, 75-90%, and even more preferably 80-90%.

Monomer units of formula (II) display on their side chain a coupling molecule R6 that permits to create a covalent bond between the copolymer according to the invention and another polymer comprising amine groups by reacting the coupling molecules R6 with these amine groups.

In the copolymer according to the invention, R6 is preferably selected from the
group consisting of: 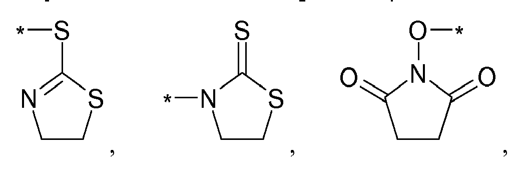 and

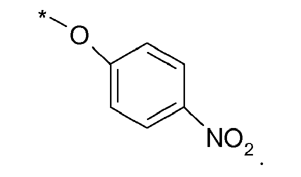

More preferably R6 is

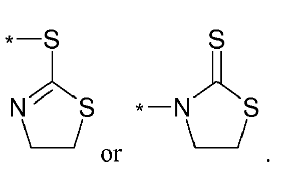

In addition, R2 is preferably selected from H and CH₃. More preferably, R2 is CH₃.

Finally, X is preferably a spacer molecule that is not pH or enzymatically cleavable in cells. Preferred examples of X include peptide GG, or any of the non-degradable spacers X defined above. A particularly preferred X is peptide GG.

Particularly preferred monomer units of formula (II) include:

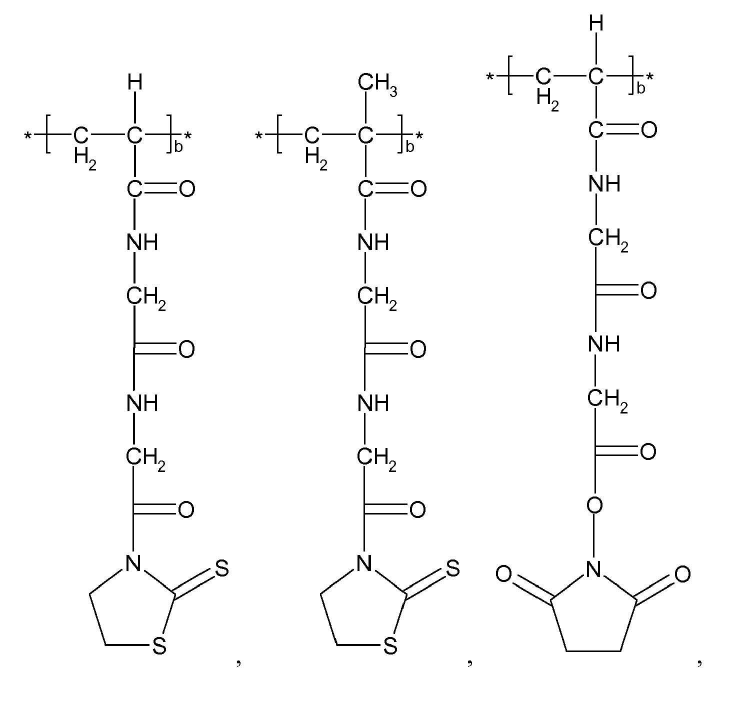

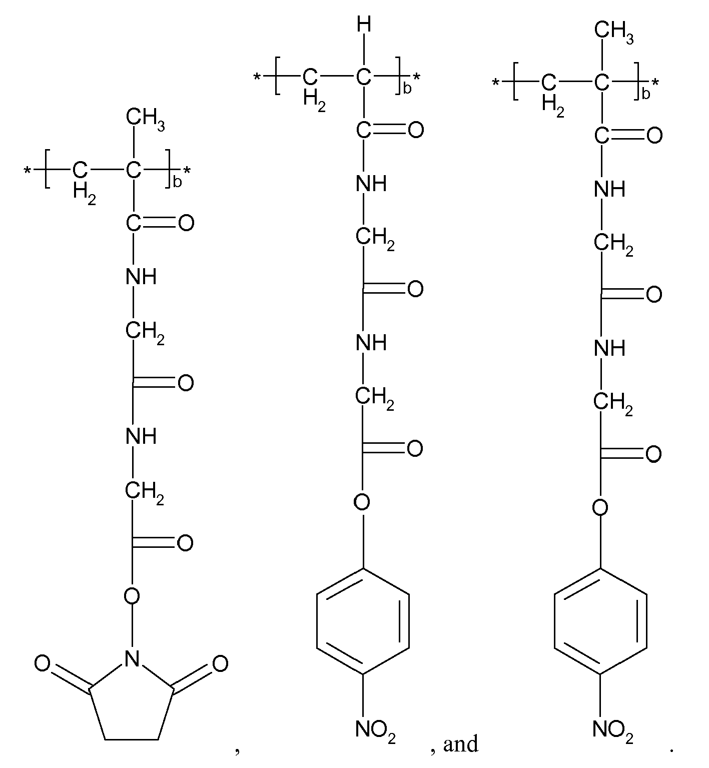

A specifically preferred monomer unit of formula (II) is:

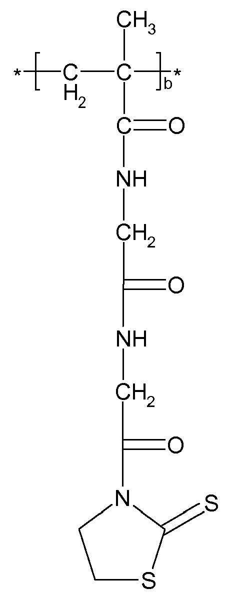

Monomer units of formula (III) display on their side chain a therapeutic agent R7 that is delivered to target cells using a nanosystem coated with the copolymer according to the invention.

Although the invention is more particularly directed to cancer treatment, since nanoparticles according to the invention comprising an external layer of the copolymer according to the invention have a size that may take advantage of the EPR effect and thus preferably accumulate in tumors, the copolymer according to the invention maybe used for other therapeutic applications. In particular, when the copolymer according to the invention comprises monomer units of formula (IV) displaying on their side chain a targeting molecule, said targeting molecule may be directed to antigens or receptors of specific non tumor cells, for instance cells from particular tissues or organs. A therapeutic agent (a drug or any inhibitor of one the cell signalitic pathway) that is beneficial for these cells in a particular disease may then be used in the copolymer according to the invention.

However, as mentioned above, in a preferred embodiment, the therapeutic agent R7 is an anticancer agent. Any suitable anticancer agent may be used with the possible necessity for chemically modifying the therapeutic agent, so that it can be coupled to the monomer by a skilled artisan. Some therapeutic agents will also need to be chemically modified and/or protected to be inert toward potential un-wanted reactions with other monomers during polymerization and nanoparticle functionalization. Examples of anticancer agents that may be used in the copolymer according to the invention include:
- Chelating agents, such as cisplatin, carboplatin, oxaliplatin, diaminedithiol, triaminethiol, tetramine, mercaptoacetyltriglycine, diethylenetriaminepentaacetic acid, 2-hydrazinonicotinic acid, N-succinimidyl-4-(18F)fluorobenzoate, 1,4,8,11-tetraazacyclotetradecane-1,4,8,11-tetraacetic acid, N-succinimidyl-5-iodo-3-pyridinecarboxylate, diethylenetriaminepentaacetic acid, 1,4,7,10-tetraazacyclodecane-1,4,7,10-tetraacetic acid, 1,4,7-triazacyclodecane-1,4,7-triacetic acid;
- Angiogenesis inhibitors, such as angiostatin K1-3, DL-a-difluoromethyl-ornithine, endostatin, fumagillin, genistein, minocycline, staurosporine, (±)-thalidomide;
- DNA intercalators or cross-linkers, such as: bleomycin, carboplatin, carmustine, chlorambucil, cyclophosphamide, cis-diammineplatinum(II) dichloride (cisplatin), melphalan, mitoxantrone, oxaliplatin;
- DNA synthesis inhibitors, such as (±)-amethopterin (methotrexate), 3-amino-1,2,4-benzotriazine 1,4-dioxide, aminopterin, cytosine β-D-arabinofuranoside, 5-fluoro-5'-deoxyuridine, 5-fluorouracil, ganciclovir, hydroxyurea, mitomycin C;
- DNA-RNA transcription regulators, such as actinomycin D, daunorubicin, doxorubicin, homoharringtonine, idarubicin;
- Enzyme inhibitors, such as S(+)-camptothecin, curcumin, (-)-deguelin, 5,6-dichlorobenz-imidazole, 1-β-D-ribofuranoside, etoposide, formestane, fostriecin, hispidin, 2-imino-1-imidazoli-dineacetic, acid (cyclocreatine), mevinolin, trichostatin A, tyrphostin AG 34, tyrphostin AG 879, inhibitors of the ribosome, inhibitors of the proteasome. Preferred enzyme inhibitors include S(+)-camptothecin, curcumin, (-)-deguelin, 5,6-dichlorobenz-imidazole, 1-β-D-ribofuranoside, etoposide, formestane, fostriecin, hispidin, 2-imino-1-imidazoli-dineacetic, acid (cyclocreatine), mevinolin, trichostatin A, tyrphostin AG 34 and tyrphostin AG 879,
- Gene regulation agents, such as 5-Aza-2'-deoxycytidine, 5-azacytidine, cholecalciferol (vitamin D3), 4-hydroxytamoxifen, melatonin, mifepristone, raloxifene, all trans-retinal (vitamin A aldehyde), retinoic acid, all trans (vitamin A acid), 9-cis-retinoic acid, 13-cis-retinoic acid, retinol (vitamin A), tamoxifen, troglitazone;
- Microtubule inhibitors, such as colchicine, dolastatin 15, nocodazole, docetaxel, paclitaxel, podophyllotoxin, rhizoxin, vinblastine, vincristine, vindesine, vinorelbine (navelbine), vinflunine ;
- Radioisotopes;
- Antisens nucleic aids or siRNAs,
- Other antitumor agents selected from the group consisting of disulfiram, 17-(allylamino)-17-demethoxygeldanamycin, 4-amino-1,8-naphthalimide, apigenin, brefeldin A, cimetidine, dichloromethylene-diphosphonic acid leuprolide (leuprorelin), luteinizing hormone-releasing hormone, pifithrin-a, rapamycin, thapsigargin, urinary trypsin inhibitor fragment (bikunin).

Preferred anticancer agents R7 useful in the copolymer of the invention include doxorubicin; daunorubicin; docetaxel or paclitaxel. More preferably, R7 is doxorubicin.

In addition, in monomer units of formula (III), R4 is preferably selected from H and CH₃. More preferably, R4 is CH₃.

Finally, therapeutic agent R7 of monomer units of formula (III) is preferably released in lysosomes of target cells. As a result, Y is preferably an enzyme-specific cleavable spacer molecule or a pH-specific cleavable spacer molecule, preferably selected from the group consisting of peptides of 2-20 amino acids comprising an amino acid sequence selected from the group consisting of FL, LF, FF, FK, VC, GFA, LALA (SEQ ID NO:1), GFLG (SEQ ID NO:2), GLFG (SEQ ID NO:3), GFFG (SEQ ID NO:4), GFYA (SEQ ID NO:5), GFAL (SEQ ID NO:6), GFLFG (SEQ ID NO:7), and YGGFL (SEQ ID NO:8). A particularly preferred Y spacer molecule is the peptide of amino acid sequence GFLG (SEQ ID NO:2).

Particularly preferred monomer units of formula (III) include those in which R4 is H and CH₃, preferably CH₃, and Y is the peptide of amino acid sequence GFLG (SEQ ID NO:2), resulting in formulas: 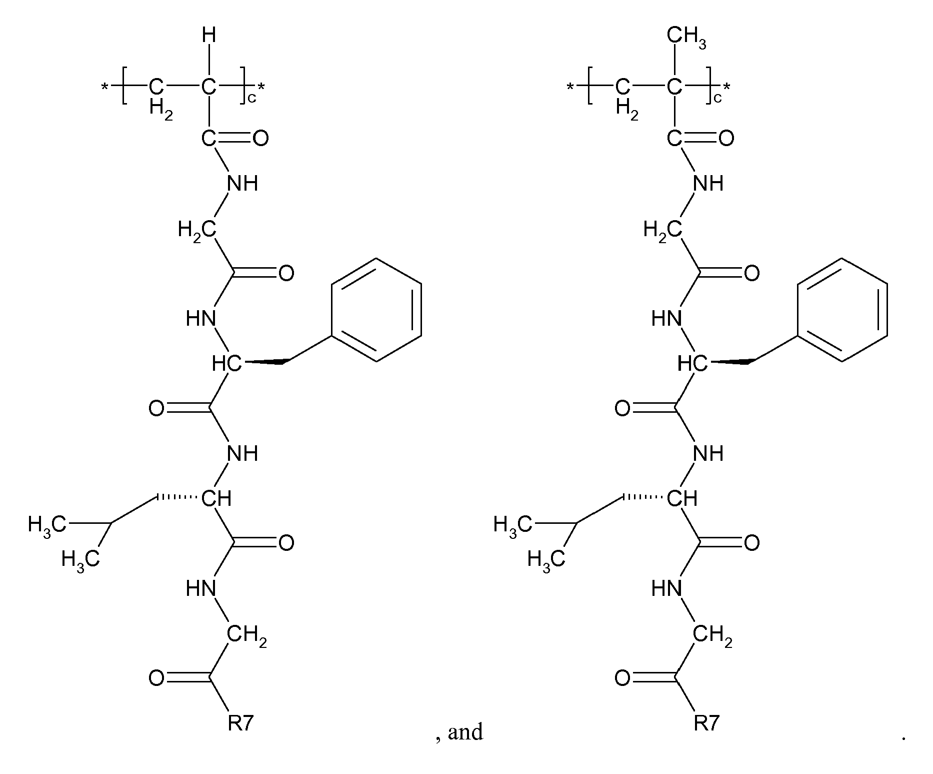 preferably 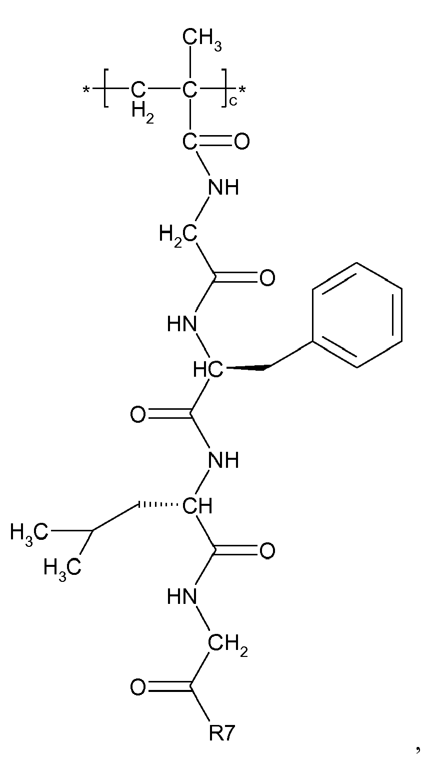 wherein R7 is the therapeutic agent as defined above, in particular an anticancer agent, notably selected from doxorubicin; daunorubicin; docetaxel or paclitaxel, preferably doxorubicin and c is as defined above.

Monomer units of formula (IV) display on their side chain a targeting molecule R8 that permits to use an active targeting of the copolymer or any material coated with the copolymer, such as a nanoparticle, to selected target cells, notably to tumor cells. The targeting molecule R8 permits to specifically target cells able to bind the selected molecule. Cells from particular tissues or organs always express tissue- or organ-specific antigens or receptors and may thus be targeted using ligands of these antigens.

In a preferred embodiment, target cells are tumor cells. Since tumor cells generally express tumor antigens that are not present on healthy cells, the use of a targeting molecule that specifically binds to a tumor antigen permits to target the copolymer of nanosystem coated by the copolymer only to tumor cells, without affecting healthy cells.

In this case, as is clearly apparent for a skilled artisan, the choice of a targeting molecule is not particularly limited in general but depends on the targeted tumor cells. Thus, depending on the type of cancer, and on the expression or not by cancer cells of known tumor antigens, a suitable targeting molecule may in each case be easily selected by a skilled artisan. Indeed, tumor antigens and specific ligands thereof have been described in the art for most cancer types. In addition, when a tumor antigen has been identified but no ligand has yet been described, it is in any case possible to use as targeting molecule an antibody specifically directed to this tumor antigen. The generation of specific antibodies is nowadays only routine procedure for skilled artisan.

Examples of targeting molecules R8 that may be used in the copolymer according to the invention include either peptide or protein targeting molecules and small organic ligands. Advantageously, as indicated above, these peptides and/or proteins are protected against unwanted reaction during copolymerisation, especially if they contain potentially reactive amino acids such as cysteines and lysines, more preferably by protecting primary amines and/or sulphur residues by protecting groups.

Advantageously, R8 is selected from the group consisting of:
a) Peptides sequences recognizing cancer cells surfaces,
b) Peptide targeting molecules optionally protected and preferably selected from :
   - a n-mer peptide (n ranged from 2 to 20 amino acids; see table 1 for the list of amino acids);
   - a RGD peptide, in particular a RGD peptide of formula C(RGD)k,
      wherein k is between 1 and 5,
   - a CREKA (SEQ ID NO: 9) peptide, in particular a CREKA peptide of formula (CREKA)m wherein m is between 1 and 5
   - a cyclic CTVALPGGYVRVC (SEQ ID NO: 10) peptide,
   - a peptide ligand for W11 proteins,
   - a peptide ligand for somatostatin receptor,
   - bomberin peptides, in particular of formula EQRLGNQWAVGHLM (SEQ ID NO: 11) or QWAVGHL (SEQ ID NO: 12),
   - peptides targeting aminopeptidase-N, integrine αvβ3 or heterodimeric transmembrane receptors,
   - peptides derived from tumor associated antigens,
   - neurotensin
   - calcitonin,

Preferred peptide or protein targeting molecules suitable for use in the invention include:
- a n-mer peptide (n ranged from 2 to 20 amino acids; see table 1 for the list of amino acids),
- a RGD peptide of formula (RGD)k and C(RGD)k, wherein k is between 1 and 5,
- a CREKA peptide of formula (CREKA)m wherein m is between 1 and 5,
- a cyclic CTVALPGGYVRVC (SEQ ID NO: 10) peptide,
- a peptide ligand for W11 proteins,
- a peptide ligand for somatostatin receptor,
- bomberin peptides of formula EQRLGNQWAVGHLM (SEQ ID NO: 11) or QWAVGHL (SEQ ID NO: 12),
- neurotensin,
- calcitonin,

Small organic ligands suitable for use in the invention include folic acid, vitamins A, B, C, D, or E, ligands for dihydrofolate reductase, galatosamine, lactose, multivalent galactose residues, fructose, glucose, glucosamine, (poly)glutamate, (poly)saccharides with degree of polymerization not exceeding 10, glycylglycine-galactosamine, 4-aminophenyl-lactose, and 6-(hexanaamino)-tris-((galactopyranosyl)-oxymethyl)methane.

Preferred small organic ligands suitable for use in the invention include folic acid, vitamins A, B, C, D, or E, galatosamine, lactose, multivalent galactose residues, fructose, glucose, glucosamine, (poly)glutamate, (poly)saccharides with degree of polymerization not exceeding 10, glycylglycine-galactosamine, 4-aminophenyl-lactose, and 6-(hexanaamino)-tris-((galactopyranosyl)-oxymethyl)methane.

In addition, when monomer units of formula (IV) are present (i.e. d>0), R3 is preferably selected from H and CH₃, and Z is preferably a spacer molecule that is not pH or enzymatically cleavable in cells. Preferred examples of Z include peptide GG, or any of the non-degradable spacers Z defined above. A particularly preferred Z is peptide GG.

Particularly preferred monomer units of formula (IV) are those in which R3 is H and CH₃, preferably CH₃, and Z is the peptide of amino acid sequence GG, resulting in formulas: 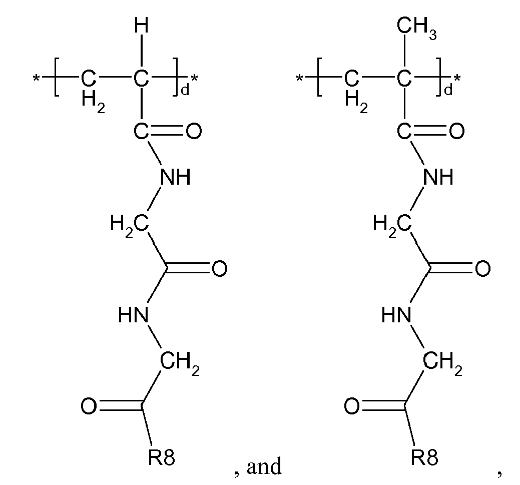 preferably 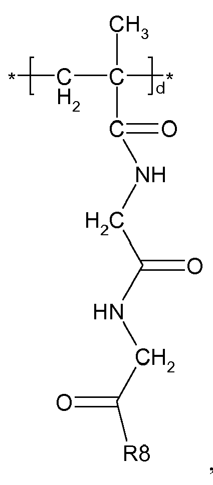 wherein R8 and d are as defined above.

However, targeting of a nanosystem coated with the copolymer according to the invention may be passive, provided that the nanosystem size is in the range for taking advantage of the EPR effect. In this case, although an active targeting may be added, it is not necessary and passive targeting may be used alone, without an additional specific active targeting.

This is the reason why the percentage d of monomer units of formula (IV) may be equal to zero in the copolymer according to the invention.

In this case, which is an advantageous embodiment according to the invention, the copolymer is a terpolymer comprising monomer units of formulas (I) (stealthiness), (II) (for covalent coupling using covalent LBL technology), and (III) (therapeutic agent).

Alternatively, passive targeting may be combined with a specific active targeting in the also advantageous embodiment in which d is strictly superior to zero, in ranges specified above. Then, the copolymer is a quaterpolymer comprising monomer units of formulas (I) (stealthiness), (II) (for covalent coupling using covalent LBL technology), (III) (therapeutic agent) and (IV) (specific active targeting).

The copolymer according to the invention is prepared by polymerization of precursors of monomer units of formulas (Ib), (IIb), (IIIb) and (IVb): 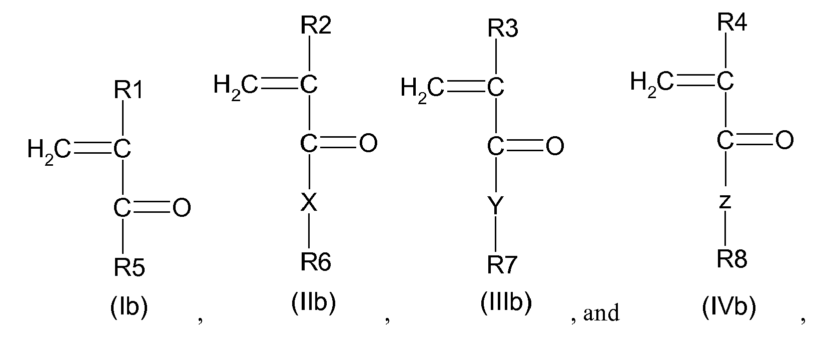 resulting after polymerization in the copolymer according to the invention comprising monomer units of formulas (I), (II), (III) and (IV).

Precursors of formula (Ib) 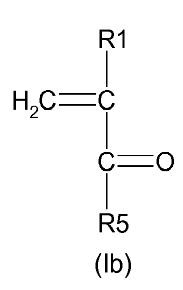 of monomer units of formula (I) may be prepared by reacting a first reagent selected from 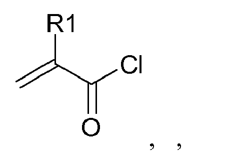 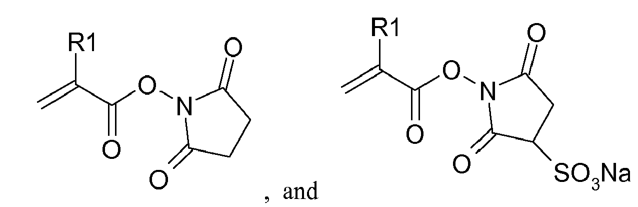 wherein R1 is as defined above with a second reagent selected from H₂N-R9, wherein R9 is as defined above; 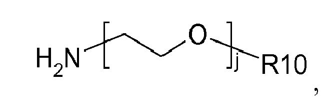 wherein R10 is as defined above; and 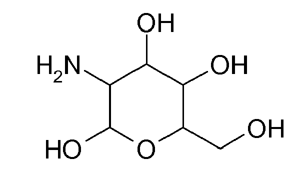 under appropriate reaction conditions that may be easily determined by any skilled artisan depending on the first and second reagents.

More precisely, when the first reagent is selected from 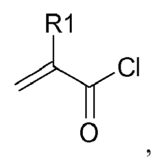 wherein R1 is H or CH₃ and the second reagent is selected from H₂N-R9, wherein R9 is CH₂-CHOH-CH₃, then both reagents may be dissolved in methylene chloride and the preparation or monomer units of formula (I) may be performed using a method comprising:
a) Mixing anhydrous sodium carbonate and the second reagent in methylene chloride to obtain a suspension,
b) Cooling the suspension to 0°C and adding dropwise and under cooling and vigorous stirring for about 1 hour a solution of the first reagent in methylene chloride.
c) Stirring the obtained reaction mixture for another 30 minutes at 15°C,
d) Adding anhydrous sodium sulfate, filtering off the solid and concentrating the dry filtrate,
e) Crystallizing monomer units of formula (I) in methylene chloride at -20°C, and purifying by recrystallization in acetone.

Precursors of formula (IIb) 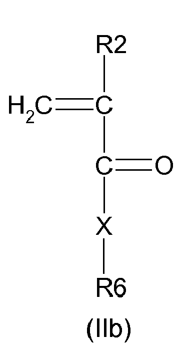 of monomer units of formula (II) may be prepared in a two steps reaction, comprising:
(1) reacting a first reagent selected from 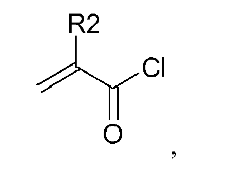 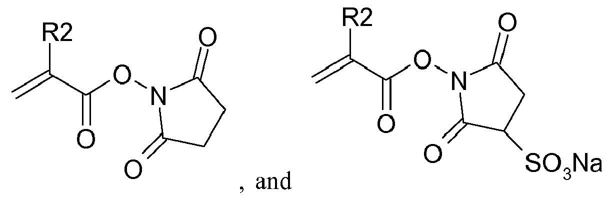 wherein R2 is as defined above with a spacer molecule X as defined above, under appropriate reaction conditions that may be easily determined by any skilled artisan depending on the selected first and second reagents, to obtain a compound of formula 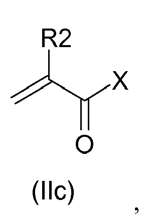 wherein X and R2 are as defined above, and
(2) reacting compound of formula (IIc) as defined above with a third reagent selected from the group consisting of 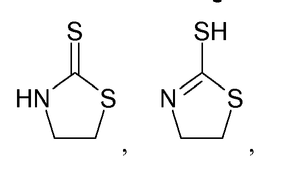 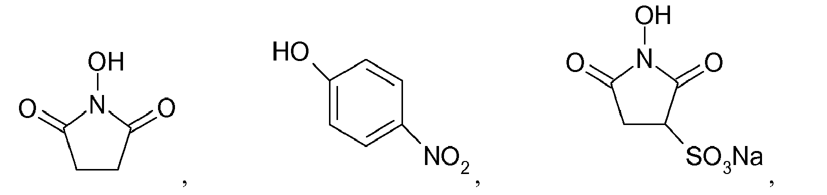 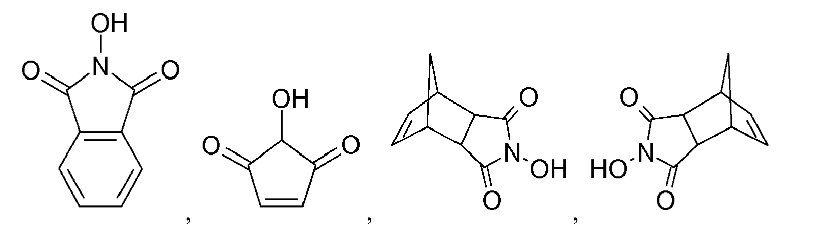 under appropriate reaction conditions that may be easily determined by any skilled artisan depending on the selected third reagent.

More precisely, when the first reagent is selected from 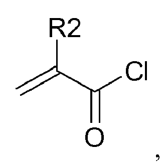 wherein R2 is as defined above, and the spacer molecule X is selected from peptides of 1-20 amino acids, then step (1) for the preparation a compound of formula (IIc) as defined above in which X is a peptide of 1-20 amino acids may be performed using a method comprising:
a) dissolving the 1-20 amino acid peptide spacer molecule X and NaOH in water,
b) simultaneously adding dropwise to the solution obtained in a) the first reagent dissolved in dichloromethane and NaOH in water under cooling and vigorous stirring for about one hour,
c) placing the reaction mixture at a temperature of about 10°C,
d) discarding the lower phase (i.e., the dichloromethane phase) and replacing it with ethyl acetate,
e) adding a concentrated HCl solution until pH < 7,
f) repeating steps d) and e)

In addition, when the third reagent is selected from 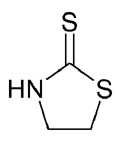 (thiazolidine-2-thione or TT), 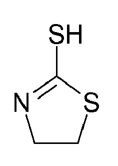 (4,5-dihydrothiazole-2-thiol), 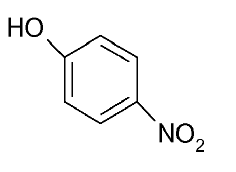 (paranitrophenol or NP), or 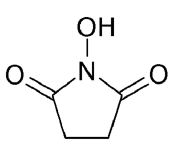 (N-hydroxysuccinimide or NHS), then step (2) for the preparation monomer units of formula (II) may be performed by reacting the compound of formula (IIc) as defined above with the third reagent in tetrahydrofuran (THF), preferably in the presence of N,N'-dicyclohexylcarbodiimide (DCC), and optionally with the addition of a catalytic amount of 4-(N,N-dimethylamino)pyridine (DMAP), or by any classical, state of the art, peptide-coupling dehydration reaction.

Precursors of formula (IIIb) 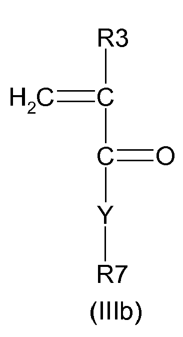 of monomer units of formula (III) may be prepared by reacting precursors of formula (IIb) with a therapeutic agent as defined above under appropriate reaction conditions, which may be easily determined by any skilled artisan depending on the therapeutic agent.

More precisely, when the therapeutic agent comprises a primary reactive amine group, precursors of formula (IIIb) may be prepared by reacting precursors of formula (IIb) in which R6 is selected from 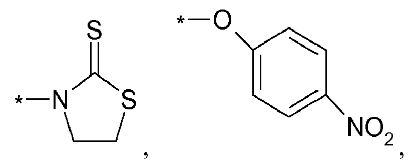 and 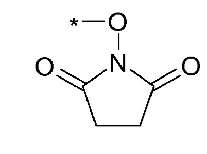 with the therapeutic agent under appropriate reaction conditions, which may be easily determined by any skilled artisan.

In particular, said precursors of formula (IIb) in which R6 is selected from 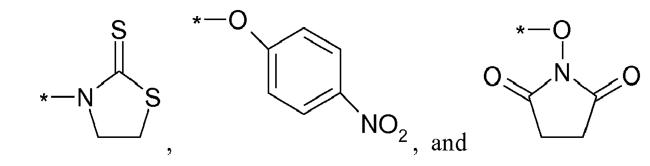 may be mixed with said therapeutic agent in DMF. Triethylamine is then added slowly under stirring, and the reaction mixture is stirred several hours at room temperature and then left in refrigerator overnight. DMF is then evaporated and diethyl ether is added to precipitate precursors of formula (IIIb).

For a therapeutic agent that does not contain a reactive primary amine group but comprises a hydroxyl group, such as paclitaxel or docetaxel, it is then possible to prepare a hemisuccinate derivative of the therapeutic agent which is further activated to the corresponding amine-reactive N-hydroxysuccinimide ester; or any other appropriate chemical reaction which will be easily determined by a skilled artisan, depending on the chemical structure of the molecule to couple and the type of spacer and monomer.

Precursors of formula (IVb) 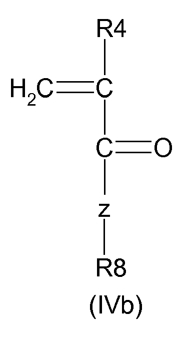 of monomer units of formula (IV) may be prepared by reacting precursors of formula (IIb) with a targeting molecule as defined above under appropriate reaction conditions, which may be easily determined by any skilled artisan depending on the targeting molecule.

More precisely, when the target molecule is a peptide or protein targeting molecule as defined above, precursors of formula (IVb) may be prepared by reacting precursors of formula (IIb) in which R6 is selected from 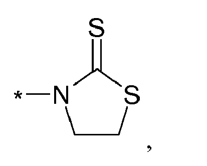 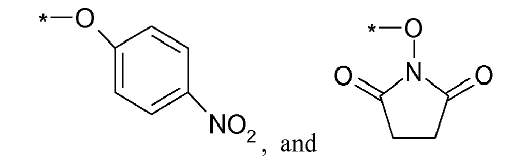 with the N-terminus of the peptide or targeting molecule under appropriate reaction conditions, which may be easily determined by any skilled artisan.

In particular, said precursors of formula (IIb) in which R6 is selected from 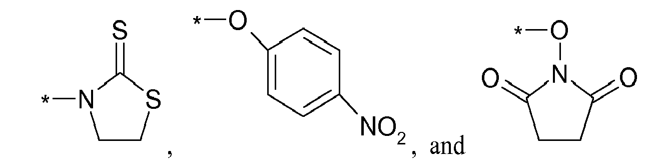 may be mixed with said peptide or protein targeting molecule in dimethylformamide (DMF). Triethylamine is then added slowly under stirring, and the reaction mixture is stirred several hours at room temperature and then left in refrigerator overnight. DMF is then evaporated and diethyl ether is added to precipitate precursors of formula (IVb).

The same method may be used for other targeting molecules comprising a reactive amine group if the targeting molecule comprises several amines, several cysteines, or several R6-reactive groups, only the one supposed to react with R6 will not be protected according the protection/deprotection strategy mentioned above.

Based on precursors of formulas (Ib), (IIb), (IIIb), and (IVb), the copolymer according to the invention may then be prepared by polymerization of these precursors in suitable conditions that may be easily determined by a skilled artisan, depending on the copolymer that is desired.

In particular, the copolymer according to the invention may be either a statistic or a block copolymer.

By a "statistic copolymer" is meant a copolymer in which monomer units of formulas (I), (II), (III), and optionally (IV) are mixed in a random manner, the only constraint being the respective percentages a, b, c and d of each monomer unit in the final monomer, which depends on the proportion of precursors of formulas (Ib), (IIb), (IIIb), and (IVb) mixed in the polymerization reaction.

Such a statistic copolymer according to the invention may be prepared by simple polymerization of a mixture of precursors of formulas (Ib), (IIb), (IIIb), and (IVb), notably using a method comprising:
a) dissolving precursors of formula (Ib) (a mol %), precursors of formula (IIb) (b mol %), precursors of formula (IIIb) (c mol %) , precursors of formula (IVb) (d mol %) and azobisisobutyronitrile (AIBN) in dimethyl sulfoxide (DMSO).
b) inserting the solution into a polymerization ampoule, bubbling the solution with nitrogen for 5 min and sealing the ampoule,
c) maintaining the ampoule at 60°C for 6 h to obtain polymerization,
d) precipitating the solution in a mixture 2:1 diethyl ether/acetone, filtering off, washing with acetone and diethyl ether,
e) dissolving the solid in methanol (10 w%), precipitating into a mixture acetone/diethyl ether (3:1), filtering off and drying in vacuum.

In such a statistic copolymer according to the invention, the degree of polymerization, and thus the molecular weight (Mw) of the obtained copolymer may be controlled by changing the % of AIBN present in the reaction mixture of above step a). A statistic copolymer according to the invention preferably has a Mw of 10³ to 10⁶, more preferably a Mw of 3.10⁴ to 6.10⁴ g/mol, which may be obtained using a % of AIBN in step a) of 5 w% to 0.001 w% (more preferably of 1 w% to 0.5 w%), with the general knowledge over the state of the art that lowering the AIBN concentration, yields polymers with higher molecular weights

By a "block copolymer" is meant a copolymer consisting of at least two distinct parts or blocks, each block comprising various percentages of the four monomer units of formulas (I), (II), (III), and (IV), the global percentage of four monomer units of formulas (I), (II), (III), and (IV) in the entire copolymer including all distinct block corresponding to a, b, c, and d respectively. Thus, in each distinct block, the percentage of monomer units of formulas (I), (II), (III), and (IV) may be distinct of a, b, c and d. In particular, one or more of monomer units of formulas (I), (II), (III), and (IV) may be absent (a, b, c, and/or d = 0) from a particular part of the copolymer, provided that at least one of monomer units of formulas (I), (II), (III), and (IV) is present. However, when taking into account all distinct parts or blocks of the block copolymer, the global percentage of monomer units of formulas (I), (II), (III), and (IV) corresponds to a, b, c and d respectively.

Such a block copolymer may be prepared by atom-transfer radical polymerization (ATRP). ATPR was first used for indirect generation of poly(HPMA) and its copolymers from a well-defined homopolymer with active ester groups such as N-hydroxysuccinimide ester groups as described above (in presence of CuBr, a typical ATRP agent; 2,2'-bipyridine; and DMSO; see Godwin, A.; Hartenstein, M.; Muller, A. H. E.; Brocchini, S. Angew. Chem. Int. Ed. 2001, 40, 594-597). Direct synthesis of well defined poly(HPMA) was also recently achieved via reversible addition fragmentation chain transfer (RAFT) polymerization (see Jia, Z. F.; Wong, L. J.; Davis, T. P.; Bulmus, V. Biomacromolecules 2008, 9, 3106-3113; Scales, C. W.; Huang, F. Q.; Li, N.; Vasilieva, Y. A.; Ray, J.; Convertine, A. J.; McCormick, C. L. Macromolecules 2006, 39, 6871-6881; Scales, C. W.; Vasilieva, Y. A.; Convertine, A. J.; Lowe, A. B.; McCormick, C. L. Biomacromolecules 2005, 6, 1846-1850; and Thomas, D. B.; Convertine, A. J.; Myrick, L. J.; Scales, C. W.; Smith, A. E.; Lowe, A. B.; Vasilieva, Y. A.; Ayres, N.; McCormick, C. L. Macromolecules 2004, 37, 8941-8950).

Thus, several subsequent polymerization reactions of type (I), (II), (III), and/or (IV) monomers, from the reactive end of the preceding polymer chain made out of monomers (I), (II), (III), and/or (IV), or any combination of those polymerized monomers may yield
- a first block with the desired proportion of monomer units of formulas (I), (II), (III), and (IV) is first prepared by polymerization of the corresponding proportion of precursors of formulas (Ib), (IIb), (IIIb), and (IVb),
- a second block is added to the first block by a second polymerization reaction with a distinct proportion of precursors of formulas (Ib), (IIb), (IIIb), and (IVb),
- if desired, further blocks may then successively be added using further successive polymerization reaction with distinct proportions of precursors of formulas (Ib), (IIb), (IIIb), and (IVb).

An example of a block copolymer according to the invention might be a four blocks copolymer, each block corresponding respectively to monomer units of formulas (I), (II), (III), and (IV), the global percentage of monomer units of formulas (I), (II), (III), and (IV) then depending on the length of each block.

In a preferred embodiment of a block copolymer according to the invention said a block copolymer comprises in any order a first block consisting of monomer units of formula (II) as defined above, linked to a second block consisting of a statistical mixture of monomer units of formulas (I), (III) and (IV) as defined above, wherein the relative percentages a, b, c and d of each monomeric unit is as defined above.

The copolymer according to the invention is particularly advantageous because it makes it possible to prepare stealthy, targeted and therapeutic nanoparticles that display several advantages compared to prior art nanoparticles as described in the background art section. In particular, the presence of an external layer of the copolymer according to the invention in a nanoparticle confers the following advantages:
- a high stability in physiological media due to the presence of monomer units of formula (I), which prevents aggregation.
- stealthiness conferred by the presence of monomer units of formula (I), permitting to avoid opsonisation by macrophages,
- an efficient drug release in target cells, thanks to the presence of the lysosomal specific cleavage of spacer Y in monomer units of formula (III) between the copolymer backbone and the therapeutic agent R7. This permits to be sure that the therapeutic agent is actually released once it has reached its target,
- a low or inexistent drug release into blood circulation, before reaching target cells. This is possible thanks to the covalent attachment of the therapeutic agent R7 to the copolymer backbone via spacer Y that is only cleaved in lysosomes.

The present invention thus further relates to a first type of core-shell nanoparticle comprising a core on which is adsorbed by electrostatic, covalent, hydrophobic or hydrogen binding a shell made of the copolymer according to the invention as defined above, wherein said nanoparticle largest dimension is in the range of 1-1000 nm, preferably 10-150 nm. In this case, a core material is simply coated with the copolymer according to the invention using various possible bindings.

Another particularly advantageous object of the present invention is a second type core-shell nanoparticle, comprising :
a) a core; and
b) a shell comprising:
   (b1) an internal shell consisting of an internal polymeric layer linked to the core by electrostatic binding and optionally one or more further internal polymeric layers, wherein each optional successive internal polymeric layer is linked to the preceding internal polymeric layer by electrostatic binding, and the last internal polymeric layer comprises amine groups,
   (b2) an external shell consisting of 1+2n external polymeric layers,
      wherein
      - n may be 0 or a positive integer,
      - each 1+2i polymeric layer, 0 ≤ i ≤ n, is made of a copolymer according to the invention as defined above, and
      - when n ≥ 1, each 2i polymeric layer, 1 ≤ i ≤ n, is a polyamine and is covalently linked to the coupling molecule of the preceding 2i-1 polymeric layer.
         wherein said nanoparticle largest dimension is in the range of 1-1000 nm, preferably 10-150 nm.

This second type core-shell nanoparticle is particularly preferred, because it displays the following further advantages compared to more simple nanoparticles only coated with the copolymer according to the invention:
- a well adjustable size thanks to the selection of a particular number of intermediate charged polyelectrolyte layers, in combination with a small polydispersity of the nanoparticle. This permits to have a well defined and modifiable size depending on the application of the nanoparticles. In particular, it permits to efficiently benefit from the EPR effect when nanoparticles are used to target cancer cells.
- a sufficient drug-loading capacity thanks to the increased area of the outer layer compared to adsorption directly onto the core. In addition, as described above, several layers of the copolymer, separated by polyamine layers to which they are covalently linked, may be added to the nanoparticle, thus permitting to further increase and adjust the drug-loading capacity. This property permits to reach sufficient drug concentration in target cells.

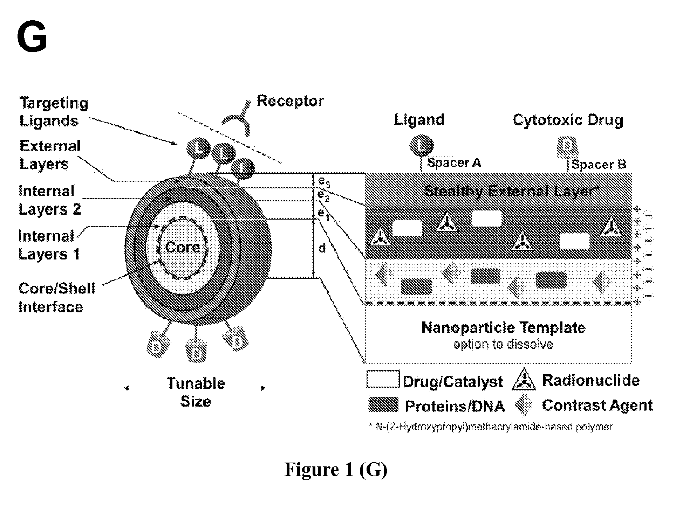

In the second type core-shell nanoparticle according to the invention, the internal shell is useful for creating a defined surface made of a polycation comprising monomers with amine groups that is independent of the core material. In order to reach this goal, the number of internal polymeric layers (alternate polycation and polyanion layers) is preferably at least 3, more preferably at least 5, or even at least 6, at least 7, at least 8, at least 9, or at least 10. The number of internal polymeric layers may further be increased to adjust the global nanoparticle size as desired and the internal shell thus has a second utility. The type of polycations and polyanions used is not really important, provided that the last internal layer is a polycation comprising monomers with amine groups. One particular motivation, however, for using a specific polyelectrolyte is its ability to retain, absorb, or couple to drugs, proteins, peptides, radionuclide, contrast agents (the later components will be inserted in the internal polyelectrolyte shells (Figure 1G). A preferred polycation is poly(allyl amine) (PAH), and a preferred polyanion is poly(styrene sulphonate) (PSS). However, these polyelectrolytes may be replaced by other polycations and polyanions. In particular, biotolerated or biodegradable polycations and polyanions may be used, in order to further increase biotolerability of the nanoparticle. Examples of polyelectrolytes (such as polycations or polyanions) are, but are not limited to, polyphosphoric acid, polyvinylsulfuric acid, polyvinylsulfonic acid, polyvinylphosphonic acid, polyacrylic acid, polyphosphate, polysulfate, polysulfonate, polyphosphonate, polyacrylate, polyethyleneimine, polyvinylamine, polyvinylpyridine. More specifically, biopolymers such as, for example, alginic acid, gum arabic, nucleic acids, pectins, oligopeptides, peptides, polypeptides, proteins, oligonucleotides, polynucleotides, DNA, RNA; and chemically modified (bio)polymers such as modified polysaccharides, for example carboxymethylcellulose, chitosan and chitosan sulfate, ligninsulfonates can be used. Other examples of polyelectrolytes include synthetic polymers (and their chemically modified counterparts) such as, for example, dendrimers, polymethacrylic acid, polyvinylsulfonic acid, polyvinylphosphonic acid, polyvinylpirolidone and polyethyleneimine, or chemically modified synthetic copolymers ofN-hydroxypropylmethacrylamide.

In the second type core-shell nanoparticle according to the invention, the external shell is useful for conferring stealthiness and thus preventing aggregation and macrophages opsonisation in physiological conditions. This property is due to the most external copolymer layer according to the invention.

In a particular embodiment of a second type core-shell nanoparticle according to the invention as defined above, said second type core-shell nanoparticle comprises only one external layer (n=0) made of the copolymer according to the invention.

Alternatively, several layers of a copolymer according to the invention, separated by polyamine layers to which they are covalently linked, may be present in the second type core-shell nanoparticle. In this case, the copolymers according to the invention used in said layers may be the same or different.

Indeed, the number of external polymeric layers may be increased by alternating the copolymer according to the invention with polyamine layers in order to increase the number of polymeric layers comprising the therapeutic agent and thus to increase the amount of therapeutic agent actually delivered to target cells.

In this case, the copolymers according to the invention used in said layers may be the same or different. In particular, the proportions of each type of monomer units may be the same or different in various external polymeric layers made of a copolymer according to the invention. In a particular embodiment, all external polymeric layers made of a copolymer according to the invention are made of the same polymer. However, in another embodiment, at least the last external polymeric layers made of a copolymer according to the invention is distinct from the others, in particular if the last external polymeric layer made of the copolymer according to the invention comprises monomer units of formula (IV) with a targeting molecule. Indeed, in external polymeric layers made of a copolymer according to the invention that are not directly in contact with the medium, the percentage d of monomer units of formula (IV) comprising targeting molecule R8 is preferably about zero since only targeting molecule that are in contact with the medium are really useful.

In any core-shell nanoparticle according to the invention as defined above -first or second type), said core is preferably made of a material selected from metals, in particular gold, silver or platinum; magnetic materials; semiconductors, in particular CdSe, ZnS, CdSe/ZnS, ZnSe or SiO₂; metal oxides; colloidal silica; silanized colloidal silica ; plastic, in particular polystyrene and melamine formaldehyde latexes; biological compounds, in particular proteins, protein-aggregates, antibodies, DNA, RNA; (polyelectrolyte/polyelectrolyte) complexes; (polyelectrolyte/multivalent ion) complexes, (polyelectrolyte/multivalent ion/metallic nanoparticles) complexes, such as so called "Nanobags" or "Micropouches", such as described in Schneider, G. F.; Decher, G. Nano Letters 2008, 8, 3598-3604.

In an advantageous embodiment, the core material is selected from metals, in particular gold, silver or platinum; magnetic materials; semiconductors, in particular CdSe, ZnS, CdSe/ZnS, ZnSe or SiO₂; metal oxides; colloidal silica; silanized colloidal silica or plastic, in particular polystyrene and melamine formaldehyde latexes. Advantageously, the core material is selected from metals, in particular gold, silver or platinum; magnetic materials; semiconductors, in particular CdSe, ZnS, CdSe/ZnS, ZnSe or SiO₂ (SiO₂ includes colloidal silica, and silanized colloidal silica)

Gold is a particularly preferred core material. Indeed, it is known not to be toxic in vivo. In addition, it further permits to add a second therapeutic utility to core-shell nanoparticles according to the invention in the case of cancer treatment. Indeed, a gold core may have been previously irradiated and thus release radiations in target tumor cells, thus adding radiotherapy to the therapeutic agent R7 released by the copolymer according to the invention in lysosomes of target cancer cells. In addition, a non irradiated gold core also permits another therapy of target cancer cells by applying radio waves to the body treated area, thus generating hyperthermia in target cancer cells comprising nanoparticles according to the inventions. Gold cores may be prepared using the standard reduction of tetrachloroauric(III) acid (HAuCl₄ · 3 H₂O) with trisodium citrate dihydrate (Na₃C₆H₅O₇ . 2 H₂O) without filtration. After their synthesis, gold cores are kept in the dark at a temperature of 5°C. Such a gold core solution is stable over more than three months.

Magnetic materials are also preferred core materials, because they also permit to generate hyperthermia in target cancer cells comprising nanoparticles according to the invention by applying magnetic waves to the body treated part. A particular example of a suitable magnetic material is maghemite Fe₂O₃. Fe₂O₃ cores may be prepared in two steps:
- synthesis of magnetite (Fe₃O₄) cores by coprecipitation of ferrous and ferric ions (1:2 stoichiometry) in an basic medium (NaOH),
- oxidation of magnetite (Fe₃O₄) cores into maghemite Fe₂O₃ cores in the presence of ferric nitrate and nitric acid, followed by magnetic decantation and, for example, redispersion in citrate or in the copolymer according to the invention (equipped with, for example, phosphated side chain groups).

Semiconductors, and notably ZnSe, are also preferred core materials, because they permit to add diagnosis ability or traceability to the nanoparticles according to the invention. These materials indeed emit luminescence and can thus be mapped in vivo. The preparation of such CdSe, ZnS, CdSe/ZnS, ZnSe or SiO₂ semiconductor core is routine procedure. More can be found in the the prior art (Bawendi, M. G.; Steigerwald, M. L.; Brus, L. E. Annual Review of Physical Chemistry 1990, 41, 477-496; Empedocles, S.; Bawendi, M. Accounts of Chemical Research 1999, 32, 389-396; Empedocles, S. A.; Neuhauser, R.; Shimizu, K.; Bawendi, M. G. Advanced Materials 1999, 11, 1243-1256; Murray, C. B.; Kagan, C. R.; Bawendi, M. G. Annual Review of Materials Science 2000, 30, 545-610; Somers, R. C.; Bawendi, M. G.; Nocera, D. G. Chemical Society Reviews 2007, 36, 579-591; Wun, A. W.; Snee, P. T.; Chan, Y.; Bawendi, M. G.; Nocera, D. G. Journal of Materials Chemistry 2005, 15, 2697-2706).

Silica is also a preferred core material, because silica core particles are very easy to prepare with a large range of sizes and very low size dispersity, thus permitting to easily and precisely adjust nanoparticles sizes to the desired application. Thus may be useful notably for the targeting of cancer cells in order to maximally benefit from the EPR effect. Preparation of monodisperse silica cores of different diameters is routine procedure, and these cores are available commercially.

(Polyelectrolyte/multivalent ion) complexes, (polyelectrolyte/multivalent ion/metallic nanoparticles) complexes are formed when a polyelectrolyte and a multivalent ion of opposite polarities are used, with or without nanoparticles (Schneider, G. F.; Decher, G. Nano Letters 2008, 8, 3598-3604). They may be prepared by mixing a polyelectrolyte with a multivalent ion of opposite polarity in the presence or absence of nanoparticles. In order to obtain flocculation and thus formation of the desired (polyelectrolyte/multivalent ion) complexes, (polyelectrolyte/multivalent ion/metallic nanoparticles) complexes, it is necessary that the total number of charges contributed by the polyelectrolyte be strictly inferior to the total number of charges contributed by the multivalent ion of opposite polarity. The size of the obtained (polyelectrolyte/multivalent ion) complexes, (polyelectrolyte/multivalent ion/metallic nanoparticles) complexes may vary from tens of nanometers to a few micrometers depending on the total concentration of the polyelectrolyte and multivalent ion in the reaction mixture. The size is decreased by decreasing the total concentration of the polyelectrolyte and multivalent ion in the reaction mixture. A well adjustable nanometer size may thus easily be obtained. In addition, when nanoparticles are present, their degree of loading on the obtained (polyelectrolyte/multivalent ion/metallic nanoparticles) complexes may be controlled by varying the stoichiometric balance between the polyelectrolyte and the multivalent ion. When the ratio between the total number of charges of the polyelectrolyte and the total number of opposite charges of the multivalent ion is decreased, the loading of nanoparticles is increased. For instance, such complexes may be obtained by mixing poly(allyl amine) (PAH) with citrate in the presence or absence of gold or iron oxide nanoparticles. In particular, complexes of 25-2000 nm size may be obtained by mixing PAH with citrate in a charge ratio of ½ and at a total citrate concentration of -20 to 200 mg/L in the reaction mixture (the concentration of PAH scales linearly with the concentration of citrate; for more details, refer to Schneider, G. F.; Decher, G. Nano Letters 2008, 8, 3598-3604). Other polyelectrolytes than PAH, and other multivalent ions can also be used to yield such nano-/micro-structures (see above for a list of polyelectrolytes of a particular interest).

The second type core-shell nanoparticles (Figure 1G) according to the invention may be prepared using a method comprising:
a) providing a core,
b) depositing onto said core an internal polymeric layer and optionally one or more further internal polymeric layers using electrostatic Layer by Layer (LBL) technology to obtain an internal shell, wherein the last internal polymeric layer comprises monomers with amine groups, advantageously wherein drugs, proteins, peptides, radionuclide, contrast agents are either chemically coupled or absorbed to the internal polymeric layers, and
c) depositing onto said last internal shell a first external polymeric layer using covalent Layer by Layer (LBL) technology, wherein said first external polymeric layer is made of the copolymer according to the invention as described above,
d) optionally repeating n times (n≥1) the following steps:
   i) depositing onto the preceding external polymeric layer made of the copolymer according to the invention a polyamine layer using covalent Layer by Layer (LBL) technology, by reacting the coupling molecules of the copolymer according to the invention with the amine groups of said polyamine to form covalent bonds, and
   ii) depositing onto the preceding polyamine layer an external polymeric layer made of the copolymer according to the invention using covalent Layer by Layer (LBL) technology, by reacting the amine groups of the preceding polyamine layer with the coupling molecules of the copolymer according to the invention to form covalent bonds,
   wherein the size of the core and the number of internal and external polymer layers are selected in order to obtain a nanoparticle largest dimension in the range of 1-1000 nm, preferably 10-150 nm.

Both electrostatic and covalent LBL technologies are well known in the art concerning flat surfaces. In addition, coating of gold nanoparticles with polyelectrolytes using electrostatic LBL technology has also been previously described (Schneider, G.; Decher, G. Langmuir 2008, 24, 1778-1789; Schneider, G.; Decher, D. Nano Letters 2004, 4, 1833-1839; Schneider, G.; Decher, G.; Nerambourg, N.; Praho, R.; Werts, M. H. V.; Blanchard-Desce, M. Nano Letters 2006, 6, 530-536), as well as the design of "Nano-bags" and "Micropouches" (Schneider, G. F.; Decher, G. Nano Letters 2008, 8, 3598-3604).

More precisely, step b) corresponding to the deposition of one or more internal polymeric layers may be performed using a method comprising:
(b1) adding a first internal polymeric layer by:
   i) dropwise adding an aqueous suspension of cores in a polyelectrolyte (polyanion or polycation) solution under vigorous stirring,
   ii) reducing agitation and incubating the reaction mixture under stirring at room temperature in the dark for at least 5 minutes, preferably 20 minutes, preferably 8-16 hours, preferably 10-14 hours, more preferably about 12 hours,
   iii) purifying the polyelectrolyte coated cores.
(b2) optionally repeating one or more times steps (b1)i) to (b1)iii) with further successive polyelectrolyte solutions,
   wherein even and odd internal polyelectrolyte layers are of opposite electric charge and the last internal polyelectrolyte layer is a polycation comprising monomers with amine groups.

Each purification step iii) may be performed using any suitable method available in the art, such as centrifugation or decantation followed by removal of supernatant, filtration, diafiltration, dialysis, ultracentrifugation and any other separation technology. In a preferred embodiment, purification is carried out by centrifugation, advantageously for about 1-3 hours (preferably about 2 hours) at 10000-15000 rpm (preferably about 13000 rpm), followed by removal of supernatant. For purification, centrifugation followed by removal of supernatant is preferably performed twice.

In the method for deposition of one or more internal polymeric layers onto the core described above, the polyelectrolyte that is used in step i) has a molecular weight adapted to the size of the core used. In fact, the molecular weight of the polyelectrolyte depends on the size of the nanoparticle used and linearly scales with the nanoparticle diameter. For instance, for cores with a diameter of 13 nm, a polyelectrolyte of about 15 000 Da is particularly suitable.

The polyelectrolyte solution used in step i) has been previously ultrasound sonicated to speed up dissolution. Advantageously, sonication may be performed in an ultrasonic bath during about one minute. This step using sonication is optional and does not influence the results.

Further deposition of 1+2n (n ≥ 0) internal polymeric layers onto the already internal shell coated cores may be performed using a method comprising:
c) depositing onto said last internal shell a first external polymeric layer made of the copolymer according to the invention by:
   i) injecting an aqueous solution of internal shell coated cores obtained in step b) into a solution of the copolymer according to the invention,
   ii) adding H₃BO₃ and Na₂B₄O₇ in order to reach a pH of about 9,
   iii) incubating the reaction mixture at room temperature in the dark for 4-8 hours, preferably about 6 hours,
d) optionally repeating n times (n ≥ 1) :
   i) purifying the copolymer coated cores obtained in step c) and redispersing them in an aqueous buffer, for example water,
   ii) injecting purified copolymer coated cores in a solution of polyamine,
   iii) adding H₃BO₃ and Na₂B₄O₇ in order to reach a pH of about 9,
   iv) incubating the reaction mixture at room temperature in the dark for 5-120 minutes, preferably about 30 minutes,
   v) purifying polyamine coated cores and redispersing them in water,
   vi) injecting purified polyamine coated cores in a solution of copolymer according to the invention,
   vii) adding H₃BO₃ and Na₂B₄O₇ in order to reach a pH of about 9,
   viii) incubating the reaction mixture at room temperature in the dark for 5-120 minutes, preferably about 30 minutes,
e) preferably aminolyzing non reacted coupling molecules R6 of the last external layer made of the copolymer according to the invention by adding an amine compound to the reaction mixture and further incubating for 0.5-2 hours (preferably about 1 hour), more preferably 1-amino-2-propanol.
f) purifying the resulting core-shell nanoparticles.

In the above method for deposition of 1+2n (n ≥ 0) internal polymeric layers onto the already internal shell coated cores, reactions are preferably carried out in plastic (for instance, polypropylene) vials or polyethylene imine (PEI) treated glass vials. Indeed, if non treated glass vials are used, the internal shell coated cores having a last internal layer comprising monomers with amine groups has a tendency to adsorb on the non treated glass surface. To prevent loss of coated cores, reactions are thus preferably performed in plastic (for instance, polypropylene) vials or poly(ethylene imine) (PEI) treated glass vials.

When coated cores are injected into a copolymer or polyamine solution in steps c)i), d)ii) or d)vi), the relative proportions of coated cores and copolymer or polyamine are preferably selected in order that for each particle, about 16.10³ to 16.10⁴ coupling molecules R6 of the copolymer according to the invention are available for reaction with amine groups of the preceding or next polymeric layer. For a copolymer having a molecular weight of 3.10⁴ to 6.10⁴ g/mol, this corresponds to about 5.10² to 5.10³ copolymer chains per nanoparticle (CPC/NP).

As in the previously described coating of cores with an internal shell, purification steps may be performed using any suitable method available in the art, such as centrifugation or decantation followed by removal of supernatant, filtration, diafiltration, dialysis, ultracentrifugation and any other separation technology. In a preferred embodiment, purification is carried out by centrifugation, advantageously for about 1-3 hours (preferably about 2 hours) at 14000-18000 g (preferably about 16000 g), followed by removal of supernatant. For purification, centrifugation followed by removal of supernatant is preferably performed two or three times. Alternatively, purification may also advantageously be performed by automated diafiltration.

The present invention further relates to a first type hollow shell nanoparticle consisting only of the shell of a second type core-shell nanoparticle according to the invention as described above. In this first type of hollow shell nanoparticle according to the invention, the shell has been separated from the core (by dissolving/etching the core), and there only remains a shell comprising an internal shell consisting of one or more internal polymeric layers linked to each other by electrostatic binding and an external shell comprising at least an external polymeric layer made of the copolymer according to the invention.

Such a first type hollow shell nanoparticle according to the invention may be prepared using a method comprising:
a) providing a second type core-shell nanoparticle according to the invention as defined above, and
b) dissolving the core.

The dissolution of the core may be performed by dipping the second type core-shell nanoparticle according to the invention in a solution easily selected by a skilled artisan for its capacity to dissolve the core material. For instance, when the core is made of gold, the core may be dissolved using potassium cyanide (KCN). Fluorhydric acid (HF) can be used for a silica core and an acidic aqueous medium (obtained with HCl for example) can be used for melamine formaldehyde latexes cores.

In a particular embodiment of a hollow shell nanoparticle according to the invention, the space left by the dissolved core may be filled with bioactive compounds, including drugs, preferably anticancerous drugs, ligands for active sites of proteins, proteins, peptides, polymers, polyelectrolytes, DNA, RNA, fluorophores, quantum dots, or radioisotopes, by modulating the porosity of the shell surrounding the core, for example using pH as described in Georgieva, R.; Moya, S.; Hin, M.; Mitlohner, R.; Donath, E.; Kiesewetter, H.; Mohwald, H.; Baumler, H. Biomacromolecules 2002, 3, 517-524.

The present invention also relates to an un-aggregated composition, comprising a plurality of un-aggregated first or second type core-shell nanoparticles according to the invention as described above or a plurality of un-aggregated hollow shell nanoparticles according to the invention as described above.

Indeed, a key feature of the core-shell or hollow nanoparticles according to the invention is their ability to remain un-aggregated in a physiological medium acceptable for injection in vivo, and further to remain un-aggregated in vivo. This is due to the presence in the copolymer according to the invention of a sufficient percentage a of monomer units of formula (I) comprising a stealthy molecule R5.

The present invention also relates to a pharmaceutical composition, comprising a first or second type core-shell nanoparticle according to the invention as defined above, a hollow shell nanoparticle according to the invention as defined above, or an un-aggregated composition according to the invention as defined above, and a pharmaceutically acceptable carrier. Any pharmaceutically acceptable carrier suitable for in vivo administration may be used. A particular suitable pharmaceutically acceptable carrier may be easily selected by a skilled artisan depending on the intended administration route, which is not particularly limited. However, in a preferred embodiment, said pharmaceutical composition may be administered directly by intravenous route in order to deliver the nanoparticles directly in the blood flow, thus permitting them to reach they target more easily and rapidly.

The present invention also relates to a first or second type core-shell nanoparticle according to the invention as described above, a hollow shell nanoparticle according to the invention as described above, an un-aggregated composition according to the invention as described above, or a pharmaceutical composition according to the invention as described above, for use as a medicament. Although said medicament may be used for delivering the therapeutic agent R7 of the copolymer according to the invention in various therapeutic applications, it is preferably intended for treating cancer, since nanoparticles according to the invention are particularly suited for benefitting from the EPR effect, thus allowing preferential accumulation in cancer cells.

Furthermore thanks to the core/shell architecture of the composition, the nanoparticles according to the present invention is particularly suited as a tumor-killing heat inducer or as a hyperthermia heat inducer when used in combination with an alternative magnetic and/or electromagnetic field in a magnetic and/or electromagnetic hyperthermia treatment. The alternative magnetic and/or electromagnetic field is generated with an appropriate device located outside the patient under treatment.

The present invention also concerns a method for delivering a therapeutic agent to target cells in vivo in a subject, comprising administering to said subject a first or second type core-shell nanoparticle according to the invention as described above, a hollow shell nanoparticle according to the invention as described above, an un-aggregated composition according to the invention as described above, or a pharmaceutical composition according to the invention as described above. In a preferred embodiment, target cells are cancer cells. Due to the advantageous properties of the nanoparticles and compositions according to the invention, the delivery of the therapeutic agent carried by the copolymer according to the invention as described above is improved since toxicity is reduced while delivery efficiency is increased.

The present invention also concerns a method for treating cancer in a subject in need thereof, comprising administering to said subject an effective amount of a first or second type core-shell nanoparticle according to the invention as described above, a hollow shell nanoparticle according to the invention as described above, an un-aggregated composition according to the invention as described above, or a pharmaceutical composition according to the invention as described above.

Another object of the present invention is a third type core-shell nanoparticle comprising :
- a core made of gold or of a magnetic material, and
- a polymeric layer made of a linear copolymer comprising monomer units of following formulas (I), (II), and (IV): 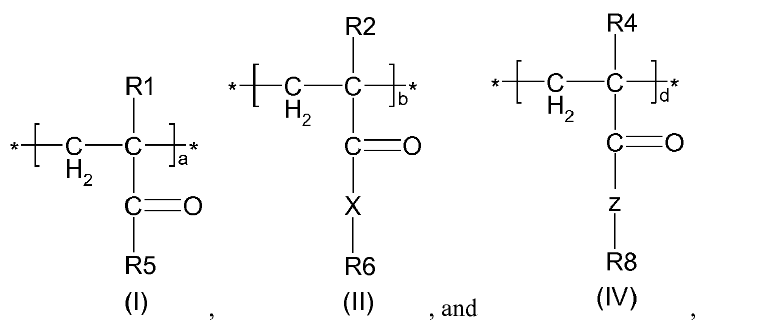
wherein R1, R2, R4, R5, R6, R8, X and Z are as defined above, and
a, b, and d correspond to the percentage of each monomer unit in the copolymer and:
a is in the range of 50-90%, preferably 80-90%,
b is in the range of 4-49%, preferably 4-19%,
d is in the range of 0-40%, preferably 0-9%, and
   preferably a + b + d is about 100%.

This object corresponds to the case in which a copolymer comprising monomer units of formula (I) with a stealthy molecule R5, monomer units of formula (II) with a coupling molecule R6, and monomer units of formula (IV) with a targeting molecule R8, corresponds to the outer layer of a core shell nanoparticle with a core made of a material permitting to confer a therapeutic anticancer utility to the core shell nanoparticle. In this case, the presence of monomer units of formula (III) with a therapeutic agent R7 is not necessary since the therapeutic ability is conferred by the core.

Indeed, as mentioned above, a nanoparticle with a gold core may be used for treating cancer by hyperthermia using radio waves to heat and destroy cancer cells having incorporated the nanoparticles due to passive or active targeting while ignoring healthy cells that have not been targeted by the nanoparticles. Similarly, a nanoparticle with a magnetic core may be used for treating cancer by magnetic hyperthermia using magnetic waves to heat and destroy cancer cells having incorporated the nanoparticles due to passive or active targeting while ignoring healthy cells that have not been targeted by the nanoparticles.

Due to the presence of monomer units of formulas (I), (II) and (IV), the other advantageous properties of the obtained nanoparticles are maintained.

In such a third type core-shell nanoparticle according to the invention, the polymeric layer made of a linear copolymer comprising monomer units of following formulas (I), (II), and (IV) may be directly adsorbed onto the core using various bindings (electrostatic, covalent, hydrophobic, hydrogen bonds), or may be the most external polymeric layer of a core-shell nanoparticle with an internal shell as defined above for second type core-shell nanoparticles and an external shell as defined above for second type core-shell nanoparticles, except that the copolymer comprising monomer units of following formulas (I), (II), (III) and (IV) is replaced by a copolymer comprising only monomer units of following formulas (I), (II), and (IV).

Still another object of the present invention is a second type hollow shell nanoparticle comprising a shell corresponding to the above described first type of hollow shell nanoparticle, in which the copolymer comprising monomer units of following formulas (I), (II), (III) and (IV) is replaced by a copolymer comprising only monomer units of following formulas (I), (II), and (IV), wherein said shell is filled with a therapeutic agent. In this case also, the presence of monomers of formula (III) comprising a therapeutic molecule R7 is not necessary. In addition, due to the presence of monomer units of formulas (I), (II) and (IV), the other advantageous properties of the obtained nanoparticles are maintained.

### DESCRIPTION OF THE DRAWINGS

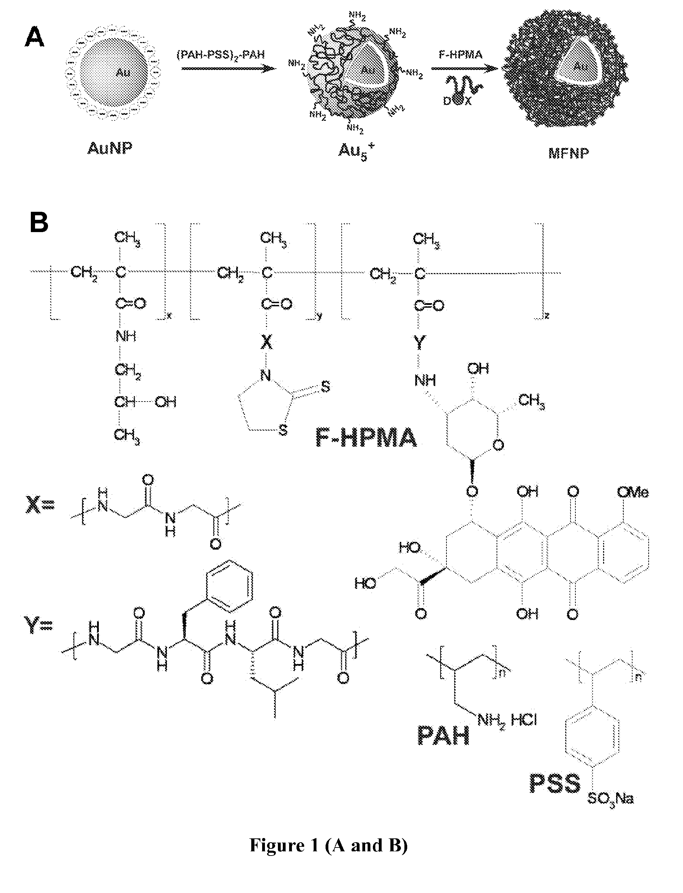

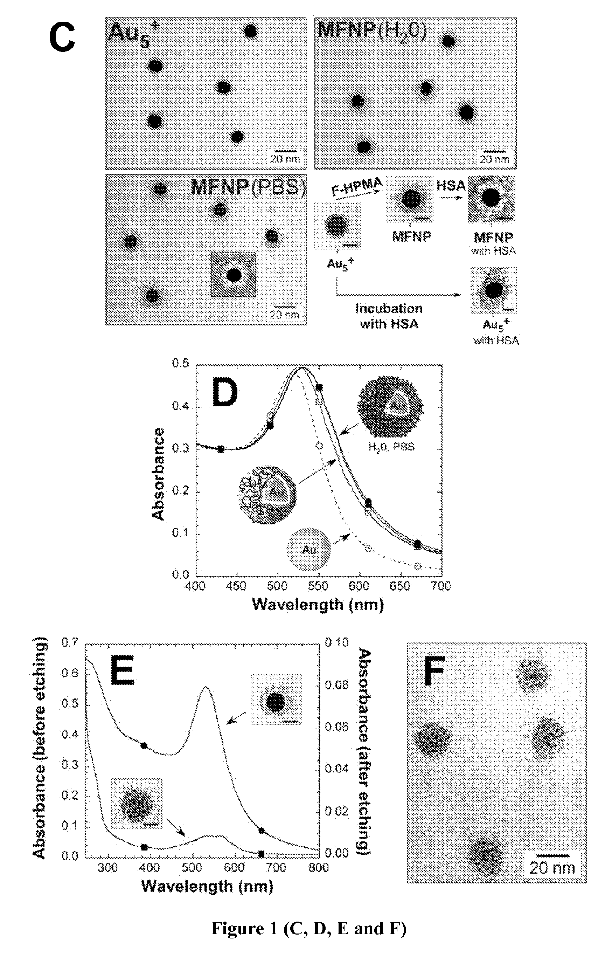

**Figure 1****. A**) From left to right: 13 nm sized gold nanoparticles (AuNPs) as obtained after synthesis, stabilised by adsorbed sodium citrate; AuNPs coated with five primer layers of PAH and PSS (i.e. Au₅₊: Au/(PAH/PSS)₂/PAH); Au₅₊ further coated with a external layer of F-HPMA (yielding MFNP). The circles in F-HPMA polymer represent doxorubicin moieties (Dox) and are at scale with respect to the size of Dox molecules and the density of Dox moieties on the nanoparticle surface. **B)** Chemical structures of the terpolymer and the polyelectrolytes used for the layer-by-layer deposition. The terpolymer F-HPMA possesses three repeating units: i) about 90 mol % of the hydrophilic backbone component N-(2-hydroxypropyl)methacrylamide (HPMA), ii) about 8 mol % of 3-(N-Methacryloylglycylglycyl) thiazolidine-2-thione (Ma-Gly-Gly-TT) for covalent LBL attachment, and iii) about 2 mol % of N-Methacryloylglycyl-DL-phenylalanylleucylglycine-doxorubicin (Ma-Gly-Phe-Leu-Gly-Dox) as active load in the form of a pro-drug. The internal compatibilisation layers, assembled by layer-by-layer deposition (LBL), are composed of poly(allyl amine hydrochloride) (PAH) and poly(styrene sulfonate) (PSS). **C)** Transmission electron micrographs of Au/(PAH/PSS)₂/PAH (Au₅⁺) dispersed in pure water, ii) Au₅⁺/F-HPMA (MFNP) dispersed in pure water, iii) MFNP dispersed in phosphate buffered saline pH 7.4 (PBS). The bottom right inset represents Au₅⁺ and MFNP, respectively dispersed in PBS in presence of 0.5 mg/mL human serum albumin (HSA). **D)** UV/Vis spectra of native AuNPs dispersed in pure water (o, dotted line), Au₅⁺ dispersed in pure water (□), MFNP dispersed in pure water (●) and in PBS (■); (●) and (■) are undistinguishable. All spectra were normalised to yield the same absorbance value at the wavelength of 440 nm. **E)** UV/Vis spectra of MFNP before (●) and after (■) the dissolution of Au-cores by an excess of KCN. Insets are TEM micrographs of MFNP before and after the dissolution of the cores. **F)** Overview TEM micrograph of four MFNPs after the etching of their Au-core (dark centers are an artefact from uranyl-acetate staining; it accumulates at the inner interface of the nanocapsule). **G)** Schematic depiction of nanoparticles coated with multilayer shells as the drug delivery system of the present invention. The multifunctionality arises from the stepwise construction of the shell that is assembled using the LBL-method. The internal layers are arbitrarily split in two compartments in order to indicate that different functionalities can be integrated in a modular way in different layers. Theoretically there is no limit with respect to the number of different internal compartments. The internal layers (1) serve primarily to compatibilize between the core and the external layers enabling the use of different core materials while maintaining the same functionalization process (they can also be reduced to zero; e.g. no internal layers). However, the internal layers may also serve to incorporate additional functional entities such as drugs, catalysts for biochemical reactions, radionuclids for radiotherapy, proteins/nucleotides for bioactivity or contrast agents for detection. The external layers carry functionalities such as enzymatically cleavable drugs or ligands for receptor mediated targeting, both of which must be accessible on the outside. Only the functionalization with an attached drug was chosen in the present study. The attachment of additional ligands, however, follows the same principle which has already been described for a variety of different functional groups in the state of the art. It is not described in this illustration, but the structure core/(internal layers)/(external layer) can be repeated several times to reach a global structure core/internal layers)/(external layer)h, where h is an unlimited positive integer higher than unity.
**Figure 2. A****)** Cathepsin B induced release of doxorubicin from MFNPs by specific cleavage of the tetrapeptide (Y=Gly-Phe-Leu-Gly) spacer between doxorubicin and the F-HPMA terpolymer backbone. The control with a Y=Gly-Gly spacer is not cleaved. **B)** Optical micrographs of TPA differentiated THP-1 leukaemia monocytes after 72 hours of incubation without nanoparticles (images on the left "THP-1" depicts the control), incubated with MFNPs (center images demonstrate the stealthiness), and incubated with Au₅⁺ (images on the right show the strong uptake of particles without F-HPMA layer).

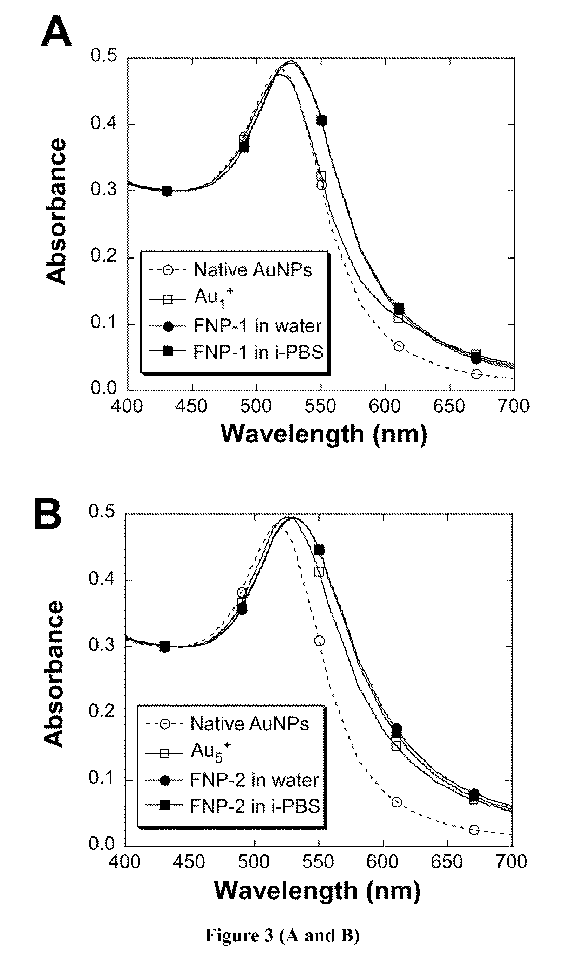

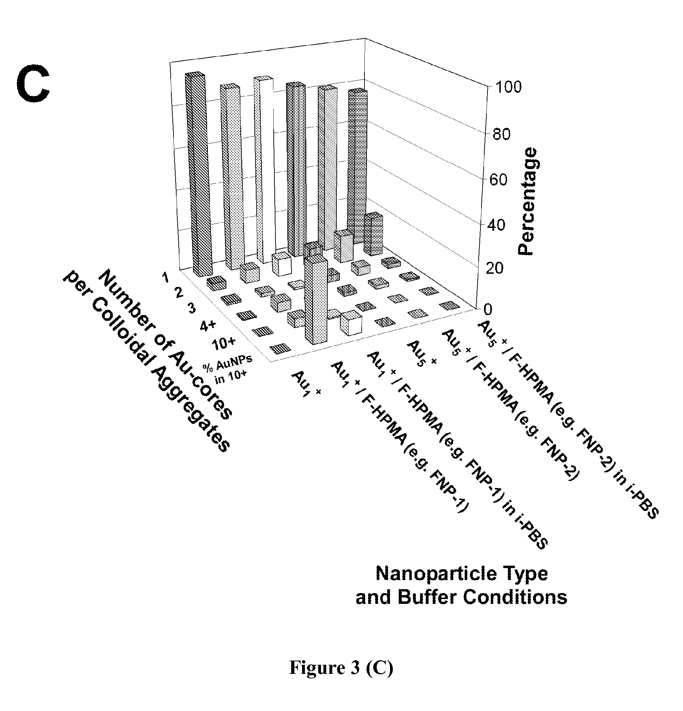

**Figure 3****.** UV/Visible spectra during the nanoparticle functionalisation for nanoparticles pre-coated with several layers of LBL-polymers and copolymer, dispersed in different buffers: **A)** Au/PAH (e.g. Au₁⁺) in pure water, Au/PAH/F-HPMA (e.g. FNP-1) in pure water, FNP-1 in isotonic PBS (i-PBS); **B)** Au/(PAH/PSS)₂/PAH (e.g. Au₅⁺) in pure water, Au/(PAH/PSS)₂/PAH/F-HPMA (e.g. FNP-2) in pure water, FNP-2 in i-PBS. Dotted lines correspond to native gold nanoparticles. All spectra were normalised in order to reach the same absorbance value at the wavelength of 440 nm. **C)** Statistical evaluation of the aggregation state derived from TEM images of 2000 Au-cores.
**Figure 4****.** Transmission electron micrographs of Au/(PAH/PSS)₂/PAH (e.g. Au₅⁺) dispersed in **A)** ultrapure water and **B)** in phosphate buffer saline, PBS. The inset in Figure 4B represents a magnified image of 1A at the place of the arrow (scale bar: 10 nm). **C)** Kinetic of Au₅⁺ aggregation monitored by UV/Visible spectroscopy and dynamic light scattering. In the inset, Rₕ represents the hydrodynamic radius.
**Figure 5****.** Chemical structures of some copolymers of the invention.
**Figure 6****.** TEM micrographs for the functionalization of Au₅⁺ with copolymer 5 (e.g. p(HPMA)-co-p(GG-TT)-co-p(GG-DOX)) for three stoichiometries: **A)** high stoichiometry (about 10000 CPCs/NP); **B)** intermediate stoichiometry (about 1000 CPCs/NP); and **C)** low stoichiometry (about 100 CPCs/NP). On average ∼1 000 copolymer chains corresponds to 32 000 TT. **D)** and **E)** Three-entries plots summarizing yields (e.g., Figure 6D) and hydrodynamic radiuses (e.g., Figure 6E) as a function of the number of copolymer chains per nanoparticle present in the reaction mixture during the functionalization of Au/(PAH/PSS)₂/PAH (e.g. Au₅⁺) with copolymers 1-8 by covalent LBL (see Figure 5 for the structure of each copolymer used). The copolymer p(HPMA)-co-p(GFLG-TT)-co-p(GFLG-Dox) is not represented here because yields of nanoparticle recovery were below 5%; that is, 95% of nanoparticles got aggregated as a pellet at the most favourable CPCs/NP.

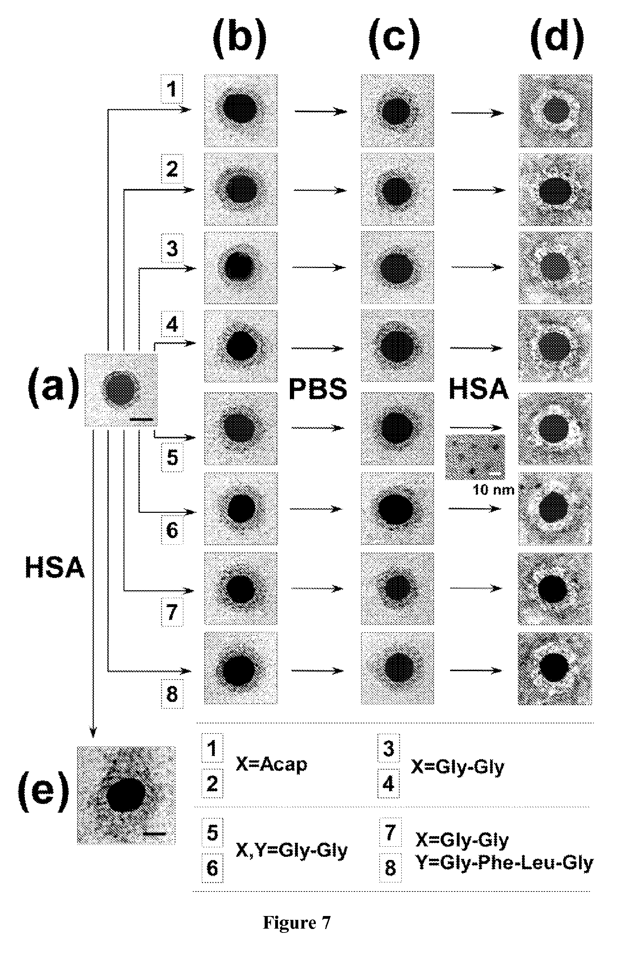

**Figure 7****. A-E)** TEM micrographs of an Au₅⁺ nanoparticle **(A)** coated with copolymers 1-8 and respectively dispersed in pure water **(B),** in PBS **(C)** or in PBS containing 0.5 mg/mL of HSA **(D).** The TEM micrograph **(E)** corresponds to Au₅⁺ incubated in presence of HSA in pure water.

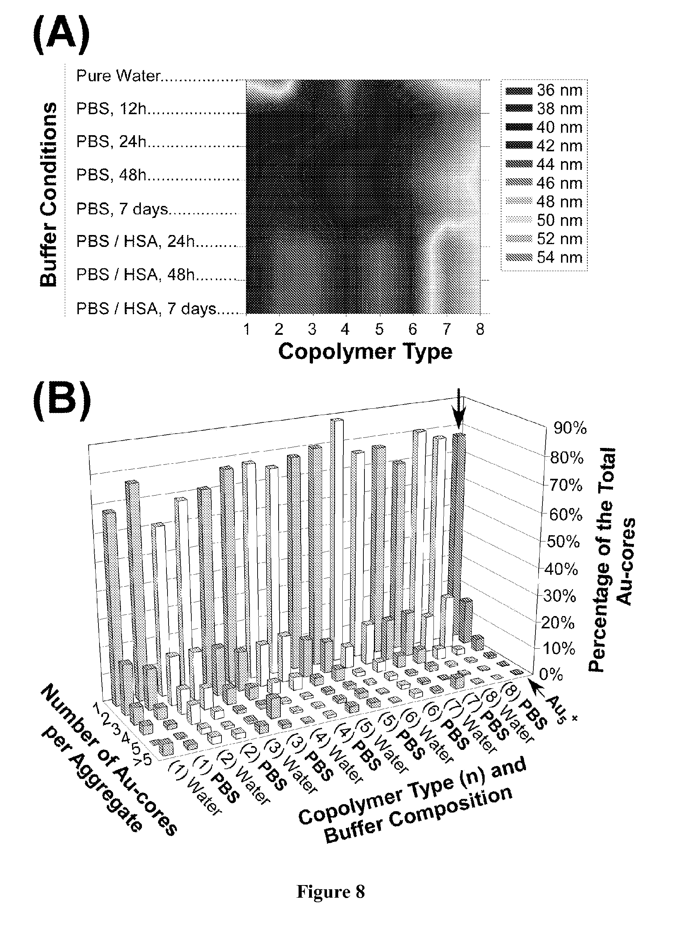

**Figure 8. A****)** Distribution of hydrodynamic radiuses for Au₅⁺/F-HPMA as a function of the buffer composition and as a function of the copolymer type. **B)** Statistical distribution by TEM of Au-cores for each type of nanoparticles, derived from copolymers 1-8.

### EXAMPLES

### EXAMPLE 1. Multifunctional Cytotoxic Stealth Nanoparticles - A Model Approach for Cancer Therapy

### 1.1. Materials and Methods

*Materials:* 1-Aminopropan-2-ol, methacryloyl chloride, glycylglycine, glycyl-L-phenylalanine, L-leucylglycine, 4-nitrophenol, 4,5-dihydrothiazole-2-thiol, 4-(dimethylamino)pyridine (DMAP), triethylamine (TEA), *N,N*-dimethylformamide (DMF), *N,N'-*dicyclohexylcarbodiimide (DCCI), 2,2'-azobisisobutyronitrile (AIBN), doxorubicin hydrochloride (Dox.HCl), Cathepsin B, ethylenediaminetetraacetic acid (EDTA), H₃BO₃, Na₂B₄O₇·10H₂O, NaCl, KCl, KH₂PO₄, Na₂HPO₄ 2H₂O, KCN and dimethyl sulfoxide (DMSO) were from Fluka AG, Buchs (Switzerland). Poly(allyl amine hydrochloride) Mw=15 000 g/mol (PAH), tetrachloroauric acid (99.9 %), trisodium citrate dihydrate, N_{α} -benzoyl-L-arginine 4-nitroanilide (Bz-Arg-Nap) and reduced glutathione were purchased by Sigma-Aldrich, hyperbranched poly(ethylene imine) (LUPASOL, M_{w}=25 000 g/mol, BASF), poly(styrene sulfonate) (PSS-pss-13k; M_{w}=13 500 g/mol) was purchased from Polymer Standard Service (PSS). THP-1 cells (ATCC, TIB-202), RPMI 1640 w/o HEPES (Invitrogen, Ref 21875-034), fetal calf serum (Eurobio), HEPES (Invitrogen, Ref 15630056), Glucose (Sigma, Ref G-7021), sodium pyruvate (Serva, Ref 15220), gentamicine (Kalys, Ref G0124-25), β-mercaptoethanol (Sigma, Ref 63690) and phorbol ester 12-O tetra decanoyl phorbol 13 acetate (TPA, Sigma).

*Synthesis of the monomers:* 1) N-(2-hydroxypropyl)methacrylamide (HPMA): the monomer was synthesised as described earlier (Ulbrich, K.; Subr, V.; Strohalm, J.; Plocova, D.; Jelinkova, M.; Rihova, B. Journal of Controlled Release 2000, 64, 63-79). 2) Ma-Gly-DL-PheLeuGly-Doxorubicin: i) The Schotten-Baumann acylation with methacryloyl chloride in an aqueous alkaline medium was employed for methacryloylation of the oligopeptide. The N-methacryloylglycyl-DL-phenylalanylleucylglycine (Ma-Gly-DL-PheLeuGly-OH) was synthesised as described earlier (Rihova, B.; Ulbrich, K.; Strohalm, J.; Vetvicka, V.; Bilej, M.; Kopecek, J.; Duncan, R. Biomaterials 1989, 10, 335-342). ii) *N*-methacryloylglycyl-DL-phenylalanylleucylglycine 4-nitrophenyl ester (Ma-Gly-DL-PheLeuGly-ONp) was prepared by the reaction of Ma-Gly-DL-PheLeuGly-OH with 4-nitrophenol in the presence of DCCI in THF as described earlier (Subr, V.; Strohalm, J.; Ulbrich, K.; Duncan, R.; Hume, I. C. Journal of Controlled Release 1992, 18, 123-132). iii) *N-*Methacryloylglycyl-DL-phenylalanylleucylglycine-doxorubicin (Ma-Gly-DL-PheLeuGly-Dox) was prepared by the reaction of 100 mg (0.172 mmol) Ma-Gly-DL-PheLeuGly-ONp with 100.5 mg (0.173 mmol) of doxorubicin hydrochloride in 2 mL of DMF. Triethylamine (26.2 µL, 0.173 mmol, 4×5.2 µL and 5.4 µL) was slowly added every ten minutes under stirring. The reaction mixture was stirred in the dark for 4 hours at room temperature and left in a refrigerator overnight. DMF was evaporated and 10 mL of diethyl ether was added. Crystals were filtered off and washed with 20 mL of diethyl ether. The powder was then dissolved in methanol (2 weight %) and purified on a Sephadex LH-20 column in methanol. 3) Ma-GlyGly-TT: *N*-methacryloylated amino acids bearing thiazolidine-2-thione reactive groups are synthesised by the reaction of *N-*methacryloylated amino acids with 4,5-dihydrothiazole-2-thiol in the presence of DCCI. Addition of catalytic amount of DMAP increased the reaction rate. The synthesis of (3-(N-Methacryloylglycylglycyl) thiazolidine-2-thione) (Ma-GlyGly-TT) is carried out as indicated in Subr, V.; Ulbrich, K. Reactive and Functional Polymers 2006, 66, 1525-1538.

*Synthesis of the F-HPMA terpolymer (e.g. p(HPMA)-co-p(GlyGly-TT)-co-p(Gly-D,L-PheLeuGly-Dox) by radical copolymerisation:* The mixture of HPMA (84 mol %, 200 mg), Ma-GlyGly-TT (12 mol %, 60.1 mg), Ma-Gly-DL-PheLeuGly-Dox (4 mol %, 65.6 mg)and 21.7 mg of AIBN (1 weight %) was dissolved in 1.82 g of DMSO (15 weight %). The solution was inserted into a polymerisation ampoule, bubbled with nitrogen for 5 min and the ampoule was sealed. Polymerisation was carried out at 60°C for 6 hours. The solution was precipitated in a mixture 2:1 diethyl ether/acetone, filtered off, washed with acetone and diethyl ether. The solid was then dissolved in methanol (10 weight %) and precipitated into a mixture acetone/diethyl ether (3:1), filtered off and dried in vacuum.

*Synthesis of Au₅⁺ and MFNPs:* The procedure yielding Au₅⁺ was inspired from our previous protocol (Schneider, G.; Decher, G. Nano Letters 2004, 4, 1833-1839.) with slight modification in order to reach a maximal amount of isolated functional nanoparticles (e.g. one Au-core per colloidal aggregate) (Schneider, G.; Decher, G. Langmuir 2008, 24, 1778-1789). Due to strong adsorption of PAH-coated AuNPs (e.g. Au₅⁺) on glass during the covalent attachment of F-HPMA, reaction flasks composed of standard glass were preliminary coated with poly(ethylene imine) (PEI, BASF-Lupasol). For that, glass flasks were filled with a mixture HCl/MeOH (1:1) (30 minutes), and replaced by pure sulphuric acid (30 minutes). They were then abundantly rinsed with ultrapure water and filled with an aqueous solution of poly(ethylene imine) (2.5 mg/mL). PEI adsorption was carried for 30 minutes. The PEI solution was then removed and flasks were intensively rinsed with ultrapure water and were dried under vacuum. A fresh aqueous stock solution of F-HPMA at 1.0 mg/mL was prepared ten minutes before use. 992 µL of this solution was mixed with ultrapure water in order to reach a final volume of 18 mL. Au₅⁺ was injected in order to reach a final absorbance in the 18 mL mixture of 0.43 (this corresponds to approximately 500 copolymer chains of F-HPMA per particles, e.g. -15 000 reactive TT groups per particles). Precisely, 10 seconds after the injection of Au₅⁺ into F-HPMA, 600 µL of H₃BO₃ (46.52 mg/mL) and 600 µL of Na₂B₄O₇, 10H₂O (69.44 mg/mL) were simultaneously added. The F-HPMA adsorption reaction was carried out for 6 hours at room temperature, in dark. After 6 hours, 6.6 µL of an aqueous solution of 1-aminopropan-2-ol (1 vol. %) is added to aminolyse un-reacted TT groups. The mixture was allowed to react for an additional hour and was then distributed in some 1.5 mL Eppendorf Safe Lock Tubes and centrifuged for 2 hours at 16 000 g and 25 °C. The supernatant was removed and replaced by ultrapure water. The centrifugation/redispersion cycle was reiterated once or twice (twice for analytical determination of the number of copolymer chains and doxorubicin per particles). After centrifugation, liquid pellets of MFNPs were collected into a glass vial (e.g. MFNPs stock solution), and the concentration in MFNPs was calculated by the measurement of the absorbance at 440 nm. This revealed a concentration in particles in the MFNP stock solution of ∼52 nmol/L.

*Characterization of the monomers, F-HPMA and nanoparticles:* Monomers were characterised by melting point, elemental analysis, NMR (Bruker, 300 MHz), HPLC (LDC Analytical) and UV/VIS spectroscopy. Purity of all monomers was examined by HPLC (LDC Analytical, USA) using a reverse-phase column Tessek SGX C18 (150 x 4 mm) with UV detection at 270 nm or 305 nm. Water-methanol containing 0.1 vol. % of TFA with gradient 50-100 vol. % methanol was used as a mobile phase; flow-rate was 0.5 mL/min. The content of 4-nitrophenyl ester or thiazolidine-2-thione reactive groups in copolymers was determined by UV spectrophotometry. The weight-average molecular weight and polydispersity of copolymers (in the case of reactive copolymers after aminolysis of reactive groups with 1-aminopropan-2-ol) were determined by SEC on Äkta Explorer HPLC (Amersham Biosciences) with Superose 6^{™} column equipped with UV, differential refractometer (Shimadzu RID-10A, Japan) and multiangle light scattering detector DAWN^{®} 8^{™} (Wyatt Technology Corp., USA). 0.3M sodium acetate buffer (pH 6.5) containing 0.5 g/L sodium azide was used as the mobile phase. The flow rate was 0.5 mL/min.

Characterisation procedure reveals: M_{w} = 56400 g/mol, M_{w}/Mₙ=1.9, TT mol % = 8.7, Dox mol % = 1.9 (6.5 molecules of doxorubicin per copolymer chain). The Dox content was determined by VIS spectrophotometry at 484 nm (ε = 13 000 Lmol⁻¹cm⁻¹) in methanol. The content of TT reactive groups was calculated from absorbance obtained by subtraction of absorbance of copolymer aminolysed with 1-aminopropan-2-ol from absorbance of copolymer containing TT reactive groups at 305 nm.

Electron micrographs were obtained with a transmission electron microscope (TEM) (Philips CM12) operating at 120 kV.

*Quantification of doxorubicin-loading per nanoparticle:* 200 µL of concentrated stock solution of MFNP (centrifuged/redispersed three times) were mixed under vigorous stirring with 100 µL of KCN (10.0 mg/mL) and 100 µL of ultra pure water for two hours to completely dissolve the gold nanocores. UV/Visible spectroscopy revealed 50.5 F-HPMA chains per MFNP after a calibration with a stock solution of F-HPMA (Figure 1E).

*Enzymatic doxorubicin-release profiles:* The enzymatic release of doxorubicine from Gly-DL-PheLeuGly-Dox side chains on MFNPs was assayed with cathepsin B. 500 mL of a buffer solution pH = 6.0 containing 250 mL KH₂PO₄ 0.1 N (3.2 g KH₂PO₄), 14.1 mL NaOH 0.1 M, 146 mg EDTA and 235.9 mL of ultrapure water (solution A) was prepared. 30 mg of reduced glutathione was disolved in 10 mL of solution A (that is the solution B). In a quartz cuvette of a path length of 10 mm, 415 µL of solution A was mixed with 500 µL of the solution B. Then, 60 µL of a solution of cathepsin B (2 mg in 500 µL) was added. The cuvette was incubated at 37 °C for 5 minutes. At this step, reduced glutathione activate the enzyme. 25 µL of a solution containing the substrate (13.93 mg of N_{α}-benzoyl-L-arginine 4-nitroanilide dissolved in 346.4 mg of DMSO) is added in the cuvette and the mixture is rapidly mixed and the absorbance at 410 nm was measured (A₄₁₀ₙₘ = 0.104). After 10 minutes of incubation at 37°C, the absorbance at 410 nm was measured again (A₄₁₀ₙₘ = 1.122). A ΔA₄₁₀ₙₘ of 0.330 for a cuvette of 10 mm path length over a time course of 10 minutes corresponds to a concentration of 2.10⁻⁷ mol/L in active enzyme. 415 µL of solution A, 500 µL of solution B, 20 µL of Cathepsin B (2.10⁻⁷ mol/L) were incubated for 5 minutes at 37°C. Then 100 µL of the MFNPs stock solution were added. The enzymatic degradation was carried out on MFNP and the released doxorubicin was analysed by HPLC equipped with TSK SGX C18 column and fluorescence detector FluoroMonitor 4100 at an excitation wavelength of 480 nm and an emission wavelength of 560 nm after 12 h, 26 h, and 36 h. Before the analysis, enzymatic reaction was stopped by freezing the mixture, colloids were removed by centrifugation. Only the supernatant was injected in the HPLC.

*In vitro incubation of Au₅⁺ and MFNPs with macrophages:* Human monocytic THP-1 cells (ATCC, TIB-202) were cultured into culture flask of 25 cm² (Greiner, Ref 690 195) in RPMI 1640 (Rosewell Parc Memorial Institut) containing 10 mM HEPES, 14 mM glucose, 1 mM sodium pyruvate, 10 % fetal calf serum, 40 µg/mL gentamicine (stock solution 200 mg/mL) and 50 µM 2-mercaptoethanol (stock solution at 100 mM). Every two days, cells were counted using a numeration Bürker cell and were diluted in supplemented RMPI in order to reach 200 000 cells/mL. After 2 days of culture, cells concentration reaches generally 700 000 cells/mL. For their differentiation in macrophages by TPA (e.g. phorbol ester 12-O tetra decanoyl phorbol 13 acetate), 1 mL of RPMI (300 000 cells) supplemented with 5 nM TPA was distributed into a 24-wells plate (Coming Incorporated 3526, Coming NY14831) in order to obtain differentiated THP-1 cells (typically 1.5 µL of a TPA solution at 1.0 mg/mL in PBS are injected in 15 mL of RPMI). The cells were incubated at 37 °C under a 5 % CO₂-humidified atmosphere for 72 hours. Then, the medium containing TPA was replaced by 500 µL of fresh RPMI medium. In 700 µL of RPMI, 14 µL of MFNPs or Au₅⁺ stock solution (both -50 nM in particles) were added and mixed. These 700 µL were then filtered under a sterile 0.22 µm Millex low binding protein filter (Millipore). 500 µL of this medium was then injected in each well containing macrophages. Every 10 minutes, over a time laps of 72 hours, optical photographs were taken from the wells with an inverted microscope equipped with a digital colour camera (Princeton, CoolSnap FX). The microscope used was a LEICA DMIRE2 used in the bright field image mode, equipped with a halogen light (12V, 100W), a shutter, an objective LEICA N PLAN 10x/0.25, a motored plate Merzhauser equipped with a temperature/CO₂-regulated box (CTI, Ref IS-M-582) controlled by electronic devices (CTI-Controller 3700 Digital for CO₂, TempControl 37-2 Digital for the temperature). After 72 hours, the medium was replaced by 1 mL of fresh RPMI and pictures were taken (Figure 2B). In wells, where nanoparticles were not internalised by macrophages (e.g. MFNPs), 500 µL of RPMI was replaced by 500 µL of RPMI containing 10 µL of Au₅⁺ (-50 nM). Time lap imaging was then continued for 72 hours revealing the further phagocytotic activity of the macrophages towards Au₅⁺.

### 1.2. Results and Discussion

In the initial experiments reported here, we designed a core/shell structure of the multifunctional nanoparticles (MFNP). First, the colloidal core composed of AuNPs was coated with five internal primer layers of poly(allyl amine) (PAH, Mw=15 000 g/mol) and poly(stryrene sulfonate) (PSS, Mw=13 500 g/mol) to create a defined polyamine surface that is independent of the core material, we denote this architecture as (PAH/PSS)₂/PAH (i.e. Au₅⁺) (Schneider, G.; Decher, G. Nano Letters 2004, 4, 1833-1839; and Schneider, G.; Decher, G. Langmuir 2008, 24, 1778-1789.). Then, a functional terpolymer (F-HPMA) was attached by covalent LBL-deposition (Decher, G. Science 1997, 277, 1232-1237; Decher, G.; Hong, J.-D.; Schmitt, J. Thin Solid Films 1992, 210/211, 831-835.; Decher, G.; Schlenoff, J. B., Eds. Multilayer thin films: Sequential assembly of nanocomposite materials; Wiley-VCH: Weinheim, 2003) to Au₅⁺ yielding the final functional core/shell MFNP nanocarrier (Figure 1A).

The terpolymer (structure presented in Figure 1B; M_{w} = 56 400 g/mol, M_{w}/Mₙ=1.9) bears three different monomer repeat units, the majority (-90 %) being hydroxypropyl methacarylamide providing for a highly water-solvated corona layer (or external layer), a prerequisite for a strong steric stabilisation and a limited opsonisation of MFNP by serum proteins. The second monomer unit is methacryloyl-glycyl-glycyl thiazolidine-2-thione (Ma-X-TT, X=GG; -8 %) to enable covalent attachment of the terpolymer to Au₅⁺ through the aminolysis of thiazolidine-2-thione reactive groups (TT) by the primary amino groups present on the surface of the PAH corona layer. The third co-monomer is methacryloyl-glycyl-phenylalanyl-leucyl-glycyl-doxorubicin (Ma-Y-Dox; -2 %) which is well known for incorporating a non-toxic pro-drug form of doxorubicin into copolymers and optimised for providing release of the active drug after endocytosis through the enzymatic degradation of the oligopeptide spacer (Y) (The approximate shell thickness revealed by TEM for Au₅⁺ is 1.5 ± 0.5 nm and ∼5.0 ± 1.0 nm for MFNPs).

The stability of (PSS/PAH)ₙ-coated NP dispersions is further enhanced by the attachment of the F-HPMA layer. While (PSS/PAH)ₙ-coated particles are stable over years in pure water, such dispersions start to aggregate when the ionic strength approaches physiological concentrations (Figure 4).

The attachment of the F-HPMA corona layer proceeds without any perturbation of the dispersion stability as evidenced by TEM micrographs (Figure 1C, H₂O) and a small displacement of the surface plasmon resonance band of the gold cores (e.g. Δλₘₐₓ < 10 nm; Figure 1D), warranting the aggregation-resistant properties of the new core/shell nanocarrier. In contrast, the attachment of the terpolymer further stabilises the particles and prevents their aggregation in simple phosphate buffered saline (PBS) at pH 7.4 or even in PBS containing human serum albumin (HSA, 0.5mg/mL, HSA was used as a model protein to monitor the opsonisation of the particles) (see Figure 1C, PBS and PBS+HSA).

In order to quantify this stabilising effect in the different media we performed a statistical evaluation of the NP aggregation (the number of Au-core present in the aggregates observed) for a total of over 2000 Au-cores (Figure 3). This analysis revealed the need for a minimum number of five internal LBL layers (n = 2) prior to the attachment of F-HPMA for keeping aggregation below 20 %, for keeping the maximum number of cores per aggregate below three and for keeping the aggregate size still below 50 nm, even if PBS is used as the dispersion medium.

A further advantage of gold NP cores is that they can easily be dissolved by KCN which causes the surfaces plasmon band to completely vanish from the spectrum (Figure 1E), resulting in the formation of non-aggregated functional nanocapsules (or hollow core-shell nanoparticles) (Figure 1F) (Schneider, G.; Decher, G. Nano Letters 2004, 4, 1833-1839.). The absence of the plasmon peak in the nanocapsule suspension allows the direct determination of the doxorubicin concentration in the sample by UV/Vis spectroscopy. After calibration with free and polymer-bound doxorubicin we calculate from the optical absorbance of the nanocapsule suspension that there is an average of about 50 F-HPMA chains adsorbed per nanoparticle. Since every F-HPMA chain carries an average of 6.5 doxorubicin molecules, this corresponds to about 325 doxorubicin moieties per particle, the UV/Vis spectra are shown in Figure IF.

The fact that doxorubicin would be released from the MFNPs surface by enzymatic clevage of the oligopeptide spacer is less than trivial considering the high density of F-HPMA chains in the corona layer. We have already demonstrated that the F-HPMA layer likely prevents the opsonisation of the nanoparticles by human serum albumine, an effect that could also hinder lysosomal enzymes (here cathepsin B) from reaching their oligopeptide target. The accessibility of the glycine-phenylalanine-leucine-glycine (GFLG) spacer and its cleavability by cathepsin B was studied by high-performance liquid chromatography (HPLC) using fluorescence detection. The results of the doxorubicin release from different MFNPs (e.g. Y=GFLG vs. Y=GG) are reported in Figure 2A. We observed that about 50 % of bound doxorubicin was released from MFNPs after 36 hours of incubation with cathepsin B. In these "static" enzymatic experiments, the enzyme is only added at time t = 0 and the known loss of the proteolytic activity of cathepsin B as a function of time was not compensated. Such a reduction of proteolytic activity never occurs after the endocytosis of nanoparticles by cells in vitro or in vivo and the values reported here can therefore be taken as minimum release values. The fact that the release of doxorubicin is entirely due to the specific cleavage by cathepsin B and not to unspecific hydrolysis was corroborated by comparison with nanoparticles coated with a corona layer in which the doxorubicin was attached to the terpolymer backbone via a non-cleavable Gly-Gly spacer (Y=GG). In this case the doxorubicin release with time was below detection (Figure 2A).

An extremely critical issue for engineering potentially long circulation times of drug carriers in vivo depends on whether the F-HPMA corona layer would render the nanoparticles "stealthy". In this study we used TPA-differentiated THP-1 monocytes as model macrophages for testing stealthiness. Au₅⁺ and MFNPs were incubated in vitro for 72 hours in the presence of THP-1 monocytes (Figure 2B). Again we took benefit from the strong extinction value of the plasmon band of the gold cores to monitor the phagocytosis of the two different nanoparticles by the THP-1 cells.

After the full incubation time, macrophages incubated with MFNPs exhibited the same pale colour as macrophages that were kept in the absence of nanoparticles. In contrast, macrophages incubated with Au₅⁺ turned dark pink/purple after only a few hours (< 6 hours) of exposure to nanoparticles. While this coloration in the presence of Au₅⁺ particles does not permit to distinguish if the particles are merely adsorbed to the surface of the macrophages or if they are truly internalised, the absence of this coloration in the cases of MFNPs (independently of n) is a clear proof that these nanoparticles are indeed stealthy: they are neither adsorbed to the macrophage surface nor internalised.

In addition, it still needs to be ruled out, that the presence of highly cytotoxic MFNPs nanoparticles (as they can potentially deliver the doxorubicin) harms or kills the THP-1 cells which may also prevent them from becoming coloured. This proof was simply established by adding Au₅⁺ particles to the macrophages after a previous incubation with MFNPs nanoparticles. The observation of a strong coloration of the THP-1 cells after addition of the Au₅⁺ particles clearly demonstrates that they were not significantly affected by the cytotoxic particles even after incubation times longer than three days.

### 1.3. Conclusions

In the present example, we show that several important demands for nanocarrier systems, which are widely thought to constitute the next generation of drugs (Ferrari, M. Nature Review Cancer 2005, 5, 161-171; Peer, D.; Karp, J. M.; Hong, S.; FaroKhzad, O. C.; Margalit, R.; Langer, R. Nature Nanotechnology 2007, 2, 751-760; Torchilin, V. P. Advanced Drug Delivery Reviews 2006, 58, 1532-1555)., can be engineered for application in cancer treatment using LBL-technology.

In particular, the combined use of both electrostatic and covalent LBL deposition onto gold nanoparticles permitted to obtain a drug delivery nanosystem with many advantages compared to known systems:
- The absence of aggregation in physiological conditions and of macrophage opsonisation ensures an increased bioavailability in vivo.
- The capacity to adjust nanoparticle size to any desired size, with a very low dispersity, as well as the optional presence of targeting molecules at the surface of the nanoparticles ensures an increased targeting of nanoparticles to cancer cells
- The attachment of the therapeutic agent to a covalently linked copolymer through a spacer that is only cleavable by lysosomal enzymes ensures reduced release of the therapeutic agent in blood circulation (i.e. decreased toxicity) and increased release in target cancer cells (i.e. increased efficiency).

There are even options to include further functionalities while maintaining the ease of the LBL-process. Most importantly, we show that the stealthing of nano-objects can be achieved with a copolymer of HPMA instead of the traditionally used poly(ethylene oxide) or dextran, which opens an entirely new possibility to engineer different multifunctional polymers for obtaining highly stable dispersions of multifunctional stealthed nanomaterials. This concept makes our new nanocarrier system a likely candidate for testing EPR-targeting which requires very good control of particle sizes.

### EXAMPLE 2. Aggregation-Resistant Multifunctional Core/Shell Nanoparticles Prepared by Electrostatic and Covalent Layer-by-Layer Assembly

### 2.1. Materials and Methods

Materials, synthesis of monomers, synthesis of the copolymers by radical copolymerisation, characterisation of monomers, and nanoparticles, synthesis of Au₅⁺ and MFNPs, quantification of doxorubicin-loading per nanoparticle, and enzymatic doxorubicin-release profiles were as described in Example 1.

### 2.2. Results and Discussion.

*State of the art: Nanoparticles stabilized with a sole electrostatic LBL process aggregate in isotonic buffers such as PBS.* We already reported efficient functionalization protocols (e.g. no aggregation and high yields) for gold nanoparticles using the Layer-by-Layer technique in pure water (Schneider, G.; Decher, G. Nano Letters 2004, 4, 1833-1839; Schneider, G.; Decher, G.; Nerambourg, N.; Praho, R.; Werts, M. H. V.; Blanchard-Desce, M. Nano Letters 2006, 6, 530-536.; Schneider, G.; Decher, G. Langmuir 2008, 24, 1778-1789). However, when incubated in phosphate buffer saline (e.g. PBS), nanoparticles strongly aggregate as a result of the screening of their charges by sodium and chloride ions (e.g. loss of electrostatic stabilization) as depicted by Figure 4 representing the behaviour of Au/(PAH/PSS)₂/PAH in PBS (abbreviated Au₅⁺, e.g. gold nanoparticles bearing five layers of polyelectrolytes; PAH: poly(allyl amine hydrochloride), PSS: poly(styrene sulfonate). This aggregating behaviour of the core/shell nanoparticles in PBS is clearly demonstrated by TEM where particles get close packed in the presence of PBS (Figure 4A-B), UV/Visible spectroscopy (Figure 4C) with a shift of the plasmon band resonance from 526.4 nm to 544.3 nm, and dynamic light scattering showing a dramatic increase of the averaged hydrodynamic radius from less than 50 nm to more than 1 µm in presence of PBS (Figure 4C, inset).

We will discuss in the next sections of the manuscript how to overcome this aggregating behaviour which is really problematic since the versatility of the LBL process can not be applied for potential therapeutic purposes when applied to nanoparticles (unless one can maybe find a polyelectrolyte that, in addition of being sufficiently charged for being adsorbed, can also confer a highly hydrated outer layer to provide an enough good steric stabilization for preventing the nanoparticles to aggregate; in the present invention, we proposed HPMA copolymers bearing coupling groups more preferably selected from NH₂ or SO₃Na, see above).

*Design of HPMA-containing functional copolymers (F-HPMA) towards their use in an optimal covalent LBL process*. In light of designing multifunctional nanoparticles for biological application, it is critical that the outer layer bears very little charges to limit salt-induced aggregation but also to limit serum protein adsorption. Therefore, the use of a truly electrostatic LBL approach for the functionalization of the last layer (e.g. the interface between the nanocarrier and a biological environment) is unlikely. Alternatively, we propose to use a covalent LBL approach where a F-HPMA copolymer will be covalently attached to the outer LBL layer by aminolysis of some thiazolidine-2-thione reactive group with primary amino groups of PAH. We opted for thiazolidine-2-thione reactive group because they are more selective toward aminolysis (as opposed to hydrolysis) than other functional groups such as, for example, paranitrophenol. 1.

The global structure of the F-HPMA copolymer proposed is shown in Figure 5. HPMA monomeric units (e.g. Ma-HPMA) are the major constituent of the copolymer. We copolymerized Ma-HPMA with some other co-monomers bearing TT groups (e.g. for the covalent association of F-HPMA with the outer PAH layer of core/shell nanoparticles) such as Ma-Acap-TT (Copolymers 1 and 2) or Ma-GlyGly-TT (Copolymers 3 and 4). For some of those copolymers (Copolymers 3 and 4), some TT groups were replaced by Doxorubicin-NH₂ HCl (Copolymers 5 and 6). Copolymers 7 and 8 represent those described in the preceding example. For each combination of monomer used, copolymers with two different molecular weight were always synthesized and referred to LMw and HMw, respectively the copolymer of a lower and higher molecular weight. The characteristics of all these copolymers are shown in following Table 2.

A such variety of copolymers were synthesized, different oligopeptidic spacers, linked or not with an hydrophobic drug, of different molecular weight, to see which combination is the most appropriate for a covalent LBL deposition (e.g. maximum drug loading, maximum yields of recovery during nanoparticle functionalization, maximal stability in physiological media and minimal serum protein adsorption).

**Table 2. Characterization of various functional copolymers**

| | | | | |
|---|---|---|---|---|
| 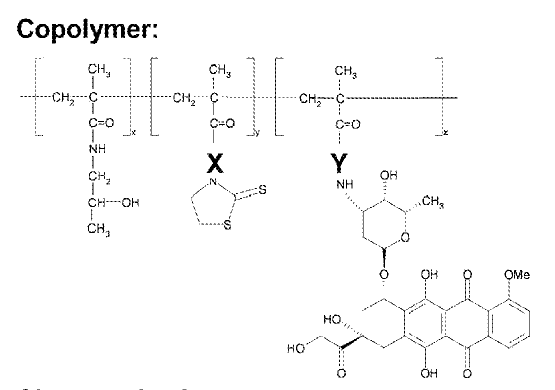 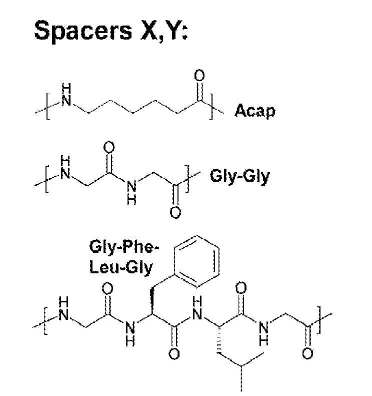 | | | | |

| **Characterization:** | | | | |
|---|---|---|---|---|
| **Copolymer Type** | **TT (%)** | **DOX (%)** | **M_{w} (g/mol)** | **M_{w}/Mₙ** |
| **(1):** p(HPMA)-co-p(Acap-TT) LMw | 8.8 | - | 33 000 | 1.8 |
| **(2):** p(HPMA)-co-p(Acap-TT) HMw | 8.5 | - | 46 000 | 1.9 |
| **(3):** p(HPMA)-co-p(GG-TT) LMw | 10.0 | - | 38 200 | 2.0 |
| **(4):** p(HPMA)-co-p(GG-TT) HMw | 10.2 | - | 53 100 | 2.1 |
| **(5):** p(HPMA)-co-p(GG-TT)-co-p(GG-DOX) LMw * | 7.3 | 1.5 | 38 200 | 2.0 |
| **(6):** p(HPMA)-co-p(GG-TT)-co-p(GG-DOX) HMw * | 7.8 | 1.5 | 53 100 | 2.1 |
| **(7):** p(HPMA)-co-p(GG-TT)-co-p(GFLG-DOX) LMw** | 9.0 | 2.0 | 44 000 | 1.6 |
| **(8):** p(HPMA)-co-p(GG-TT)-co-p(GFLG-DOX) HMw** | 8.7 | 1.9 | 56 400 | 1.9 |

| | | | | |
|---|---|---|---|---|
| * Prepared from copolymers 3 and 6 by aminolysis of some TT groups with reactive DOX-NH2 ** prepared by copolymerization of Ma-HPMA, Ma-GG-TT, Ma-GFLG-TT | | | | |

*Functionalization of Au₅⁺ nanoparticles with F-HPMA by covalent LBL: Yields of recovery, average hydrodynamic radius and morphologies*. With each copolymer described previously, we proceeded to the coating of Au₅⁺. The stoichiometry of the copolymer (e.g. the number of TT groups per particles; on average 32 000 TT groups correspond to 1 000 copolymer chains; we abbreviated respectively CPCs/NP the number of copolymer chains per nanoparticle and TTs/NP the number of TT groups per nanoparticle) was varied from 1 to 25 000 CPCs/NP (e.g. 30 to 800 000 TTs/NP) and proceeded to the evaluation of the yields of nanoparticle recovery (by UV/Vis absorbance on the plasmon band of gold NPs) and of the hydrodynamic radius (by dynamic light scattering).

For each copolymer, highest yields are observed for highest stoichiometries. However, one must consider that too high stoichiometries also yield aggregated nanoparticles, such as what we previously reported when a sole electrostatic LBL deposition is performed. This aggregation might originate from a competition between a covalent adsorption (by aminolysis) and electrostatic adsorption through the apparition of carboxylates (negatively charged) through the hydrolysis of TT group by water.

Typical stoichiometry-dependant morphologies of Au₅⁺/F-HPMA conjugates are shown in Figure 6 when copolymer 5 is used. While at low CPCs/NP (e.g. ∼100 CPCs/NP) nanoparticles aggregates into necklace-like structures (Figure 6C), at high CPCs/NP (e.g. 10000 CPCs/NP) floc-like morphologies appear (Figure 6A) (see reference Schneider, G. F.; Decher, G. Nano Letters 2008, 8, 3598-3604, for more details on floc-like morphologies). Therefore, stoichiometry has to be carefully optimized/adjusted to yield the decoration of single gold cores with F-HPMA (Figure 6B).

Concerning sizes, lowest sizes are obtained generally with intermediate stoichiometries. For instance, for copolymers 5 and 6, lowest sizes are obtained for CPCs/NP values comprised between 500 and 5 000.

Also, the optimal stoichiometry is strongly dependant on the copolymer structure, such that when more hydrophobic spacers are used (such as amino caproic acid, e.g. Acap), a larger excess of copolymer is also needed, probably due to the fact that, with more hydrophobic spacer, the TT groups are buried in a sort of hydrophobic core in the copolymer and are therefore less reactive. At -500 CPCs/NP all copolymers yielded un-aggregated Au₅⁺/F-HPMA nanoparticles (e.g. -50 nm) with acceptable yields (80-90%), except for copolymers 1 and 2 (e.g., Acap derivatives) which required slightly higher stoichiometries to reach a ∼80% yield of recovery (at -5000 CPCs/NP). Those stoichiometries (e.g. 500 and 5 000 CPCs/NP, respectively for copolymers 3-8 and 1-2) were therefore chosen for further stability investigations.

*The outer F-HPMA layer provides an homogeneous coating, a drastic stability in PBS, and an invisibility mask for serum proteins*. We investigated the properties of the covalent LBL coating (e.g. homogeneity of the F-HPMA coating, stability towards PBS and HSA) by means of TEM (Figure 7), dynamic light scattering (Figure 8A) and statistical evaluation of the number of Au-cores per colloidal aggregate (Figure 8B) (we removed un-adsorbed copolymers by centrifugation prior investigations). One can observe on individual TEM micrographs (Figure 7A-D) that the properties of the coating does not depend either on the copolymer structure used or on the nature of the dispersing medium since TEM micrographs are undistinguishable, independently of the chemical nature of the copolymer used (the absence of an F-HPMA coating clearly induce the adsorption of a layer of HSA at least twice thicker than the (PAH/PSS)₂/PAH initial layer; see Figure 7E).

We looked in more details into the properties of the dispersion of these Au₅⁺/F-HPMA nanoparticles by means of hydrodynamic radiuses using dynamic light scattering. While PBS seems to disaggregate nanoparticles coated with copolymers 1 and 2 (also corroborated by Figure 8B showing an increase in the percentage of single core colloids), for copolymers 3 to 8 no sensible variation of the hydrodynamic radius occurs (< 10 nm), whether the nanoparticles are dispersed in pure water, PBS or HSA (with times of incubation up to 7 days).

### 2.3 Conclusions.

We report here on a model approach for designing multifunctional nanoparticles that are stable in PBS and which do not associate with serum albumin. This approach of combining electrostatic and covalent Layer-by-Layer assembly yield a modular design of core/shell nanoparticles carrying pro-drugs in their polymeric shell. These nanoparticles possess two properties by which the therapeutic width of the attached doxorubicin can be enhanced:
a) A very well defined particle size, which can precisely be adjusted by synthetic procedures,(Frens, G. Nature Physical Science 1973, 241, 20-22; Turkevitch, J.; Stevenson, P. C.; Hillier, Journal Discussion Faraday Society 1951, 11, 55-75) and
b) Attachment of the doxorubicin via oligopeptide spacers designed to be cleaved by lysosomal enzymes.

While the first property permits enhanced targeting via the EPR effect, the second property permits to reduce the extracellular concentrations of the active agent.

We construct the new nanoparticles from three ingredients, a core of a certain size, here gold particles with a diameter of 13.5 nm, a few primer (or internal) layers that are deposited using electrostatic layer-by-layer assembly and a corona (or external) layer which provides for steric stabilisation, stealthiness and cytotoxicity and which is attached by covalent layer-by-layer assembly.

The approach is modular as it allows in principle to vary all of the three ingredients independently. This way one could imagine using drugs or combinations of drugs even on colloids of different sizes in order to target different diseases while maintaining a single process for the fabrication of the different particle systems.

### BIBLIOGRAPHY

US 7,101,575
US2002/0192814
US 2006/0275250
Orain, D.; Ellard, J.; Bradley, M. Journal of Combinatorial Chemistry 2002, 4, 1-16; Schelhaas, M.; Waldmann, H. Angewandte Chemie-International Edition 1996, 35, 2056-2083).
Rüegg, U.T.; Jarvis, D.; Rudinger J. Biochemical Journal 1979, 179, 127-134
Schelhaas, M.; Waldmann, H. Angewandte Chemie-International Edition 1996, 35, 2056-2083
Godwin, A.; Hartenstein, M.; Muller, A. H. E.; Brocchini, S. Angew. Chem. Int. Ed. 2001, 40, 594-597).
Jia, Z. F.; Wong, L. J.; Davis, T. P.; Bulmus, V. Biomacromolecules 2008, 9, 3106-3113
Scales, C. W.; Huang, F. Q.; Li, N.; Vasilieva, Y. A.; Ray, J.; Convertine, A. J.; McCormick, C. L. Macromolecules 2006, 39, 6871-6881
Scales, C. W.; Vasilieva, Y. A.; Convertine, A. J.; Lowe, A. B.; McCormick, C. L. Biomacromolecules 2005, 6, 1846-1850
Thomas, D. B.; Convertine, A. J.; Myrick, L. J.; Scales, C. W.; Smith, A. E.; Lowe, A. B.; Vasilieva, Y. A.; Ayres, N.; McCormick, C. L. Macromolecules 2004, 37, 8941-8950).
Schneider, G. F.; Decher, G. Nano Letters 2008, 8, 3598-3604.
Bawendi, M. G.; Steigerwald, M. L.; Brus, L. E. Annual Review of Physical Chemistry 1990, 41, 477-496.
Empedocles, S.; Bawendi, M. Accounts of Chemical Research 1999, 32, 389-396.
Empedocles, S. A.; Neuhauser, R.; Shimizu, K.; Bawendi, M. G. Advanced Materials 1999, 11, 1243-1256.
Murray, C. B.; Kagan, C. R.; Bawendi, M. G. Annual Review of materials Science 2000, 30, 545-610.
Somers, R. C.; Bawendi, M. G.; Nocera, D. G. Chemical Society Reviews 2007, 36, 579-591.
Wun, A. W.; Snee, P. T.; Chan, Y.; Bawendi, M. G.; Nocera, D. G. Journal of Materials Chemistry 2005, 15, 2697-2706.
G. Schneider, G. Decher, Nano Letters 2004, 4, 1833-1839.
G. Schneider, G. Decher, Langmuir 2008, 24, 1778-1789.
G. Decher, J. B. Schlenoff Multilayer Thin Films: Sequential Assembly of Nanocomposite Materials 2003 (Wiley-VCH, Weinheim).
Schneider, G.; Decher, G.; Nerambourg, N.; Praho, R.; Werts, M. H. V.; Blanchard-Desce, M. Nano Letters 2006, 6, 530-536.
Georgieva, R.; Moya, S.; Hin, M.; Mitlohner, R.; Donath, E.; Kiesewetter, H.; Mohwald, H.; Baumler, H. Biomacromolecules 2002, 3, 517-524.
Ulbrich, K.; Subr, V.; Strohalm, J.; Plocova, D.; Jelinkova, M.; Rihova, B. Journal of Controlled Release 2000, 64, 63-79.
Rihova, B.; Ulbrich, K.; Strohalm, J.; Vetvicka, V.; Bilej, M.; Kopecek, J.; Duncan, R. Biomaterials 1989, 10, 335-342.
Subr, V.; Strohalm, J.; Ulbrich, K.; Duncan, R.; Hume, I. C. Journal of Controlled Release 1992, 18, 123-132.
V.Subr, K.Ulbrich, Reactive and Functional Polymers 2006, 66, 1525-1538.
Decher, G. Science 1997, 277, 1232-1237.
Decher, G.; Hong, J.-D.; Schmitt, J. Thin Solid Films 1992, 210/211, 831-835.
M. Ferrari, Nat. Rev. Cancer 2005, 5, 161-171.
D. Peer, J. M. Karp, S. Hong, O. C. FaroKhzad, R. Margalit, R. Langer, Nature Nanotechnology 2007, 2, 751-760.
V. P. Torchilin, Adv. Drug. Deliver. Rev. 2006, 58, 1532-1555.
Frens, G. Nature Physical Science 1973, 241, 20-22.
Turkevitch, J.; Stevenson, P. C.; Hillier, Journal Discussion Faraday Society 1951, 11, 55-75.

## Claims

1. A linear copolymer comprising monomer units of following formulas (I), (II), (III) and (IV): 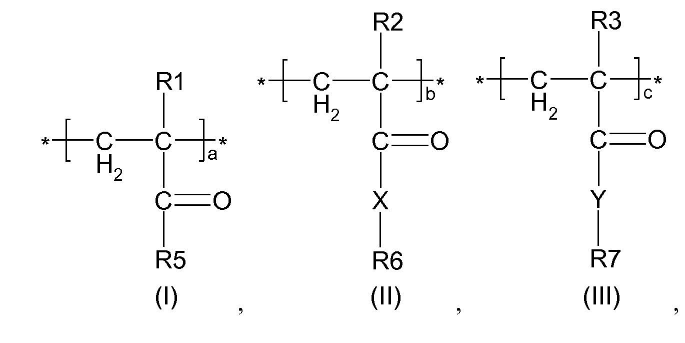 and 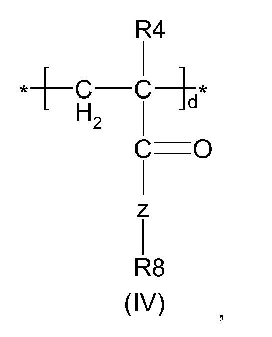 wherein
R1, R2, R3 and R4 are each independently selected from H and CH₃,
X, Y, and Z are spacer molecules independently selected from the group consisting of a peptide of 1-20 amino acids, an oligonucleotide of 1-20 nucleotides, a C₁-C₂₀ alkyl, and -(CH₂CH₂O)ᵢ wherein i is an integer in the range of 1-20;
R5 represents a stealthy molecule selected from the group consisting of:
- NHR9 wherein R9 is a hydroxylated C₁-C₆ alkyl; 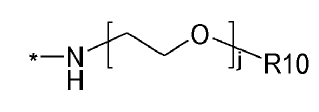
wherein R10 is selected from the group
consisting of a hydrogen atom and a hydroxylated C₁-C₆ alkyl, and 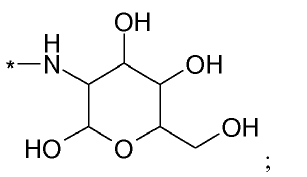 R6 represents a coupling molecule selected from the group consisting of:
-SH, -NH₂, BOC and FMOC amines, -COOH, -SO₃Na, -S-S-R11, -O-PO₃²⁻, polyphosphate, -(-(PO₄⁻)ₙ-R11, -N⁺(CH₃)₂-R11, -O-Si(OR11)₃, 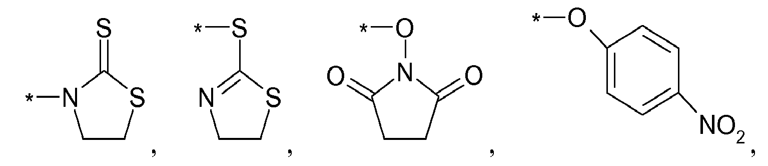 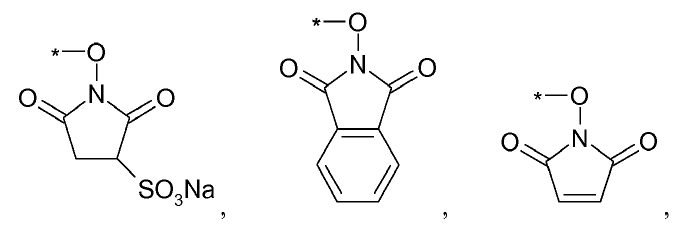 and 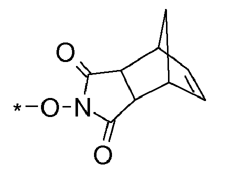
wherein R11 is selected from the group consisting of a hydrogen atom, a C₁-C₁₀ alkyl and a hydroxylated C₁-C₆ alkyl;
R7 is a therapeutic agent,
R8 is a targeting molecule, and
a, b, c and d correspond to the percentage of each monomer in the copolymer and:
a is in the range of 50-90%, preferably 80-90%,
b is in the range of 4-49%, preferably 4-19%,
c is in the range of 1-40%, preferably 1-10%,
d is in the range of 0-40%, preferably 0-9%, and
preferably a + b + c + d = 100%,
and * indicates the position involved in binding with another group, and

2. The copolymer according to claim 1, wherein Y is an enzyme-specific cleavable spacer molecule or a pH-specific cleavable spacer molecule, preferably selected from the group consisting of peptides of 2-20 amino acids comprising an amino acid sequence selected from the group consisting of FL, LF, FF, FK, VC, GFA, LALA (SEQ ID N0:1), GFLG (SEQ ID NO:2), GLFG (SEQ ID NO:3), GFFG (SEQ ID NO:4), GFYA (SEQ ID NO:5), GFAL (SEQ ID NO:6), GFLFG (SEQ ID NO:7), and YGGFL (SEQ ID NO:8).

3. The copolymer according to anyone of claims 1-2, wherein said targeting molecule R8 is selected from the group consisting of:
a) peptides sequences recognizing cancer cell surface
b) peptide targeting molecules optionally protected, preferably selected from:
- a n-mer peptide, n ranged from 2 to 20 amino acids,
- a RGD peptide, in particular a RGD peptide of formula C(RGD)k,
wherein k is between 1 and 5,
- a CREKA (SEQ ID NO: 9) peptide, in particular a CREKA peptide of formula (CREKA)m wherein m is between 1 and 5
- a cyclic CTVALPGGYVRVC (SEQ ID NO: 10) peptide,
- a peptide ligand for W11 proteins,
- a peptide ligand for somatostatin receptor,
- bomberin peptides, in particular of formula EQRLGNQWAVGHLM (SEQ ID NO: 11) or QWAVGHL (SEQ ID NO: 12),
- peptides targeting aminopeptidase-N, integrine αvβ3 or heterodimeric transmembrane receptors,
- peptides derived from tumor associated antigens,
- neurotensin
- calcitonin,
c) small organic ligands, preferably selected from folic acid, vitamines A, B, C, D, or E, ligands for dihydrofolate reductase, galatosamine, lactose, multivalent galactose residues, fructose, glucose, glucosamine, (poly)glutamate, (poly)saccharides with degree of polymerization not exceeding 10, glycylglycine-galactosamine, 4-aminophenyl-lactose, and 6-(hexanaamino)-tris-((galactopyranosyl)-oxymethyl)methane.

4. The copolymer according to anyone of claims 1-3, wherein said therapeutic agent R7 is an anticancer drug, preferably derived from the group consisting of:
- Chelating agents, such as cisplatin, carboplatin, oxaliplatin, diaminedithiol, triaminethiol, tetramine, mercaptoacetyltriglycine, diethylenetriaminepentaacetic acid, 2-hydrazinonicotinic acid, N-succinimidyl-4-(18F)fluorobenzoate, 1,4,8,11-tetraazacyclotetradecane-1,4,8,11-tetraacetic acid, N-succinimidyl-5-iodo-3-pyridinecarboxylate, diethylenetriaminepentaacetic acid, 1,4,7,10-tetraazacyclodecane-1,4,7,10-tetraacetic acid, 1,4,7-triazacyclodecane-1,4,7-triacetic acid;
- Angiogenesis inhibitors, such as angiostatin K1-3, DL-a-difluoromethyl-ornithine, endostatin, fumagillin, genistein, minocycline, staurosporine, (±)-thalidomide;
- DNA intercalators or cross-linkers, such as: bleomycin, carboplatin, carmustine, chlorambucil, cyclophosphamide, cis-diammineplatinum(II) dichloride (cisplatin), melphalan, mitoxantrone, oxaliplatin;
- DNA synthesis inhibitors, such as (±)-amethopterin (methotrexate), 3-amino-1,2,4-benzotriazine 1,4-dioxide, aminopterin, cytosine β-D-arabinofuranoside, 5-fluoro-5'-deoxyuridine, 5-fluorouracil, ganciclovir, hydroxyurea, mitomycin C;
- DNA-RNA transcription regulators, such as actinomycin D, daunorubicin, doxorubicin, homoharringtonine, idarubicin;
- Enzyme inhibitors, such as S(+)-camptothecin, curcumin, (-)-deguelin, 5,6-dichlorobenz-imidazole, 1-β-D-ribofuranoside, etoposide, formestane, fostriecin, hispidin, 2-imino-1-imidazoli-dineacetic, acid (cyclocreatine), mevinolin, trichostatin A, tyrphostin AG 34, tyrphostin AG 879, inhibitors of the ribosome, inhibitors of the proteasome.
- Gene regulation agents, such as 5-aza-2'-deoxycytidine, 5-azacytidine, cholecalciferol (vitamin D3), 4-hydroxytamoxifen, melatonin, mifepristone, raloxifene, all trans-retinal (vitamin A aldehyde), retinoic acid, all trans (vitamin A acid), 9-cis-retinoic acid, 13-cis-retinoic acid, retinol (vitamin A), tamoxifen, troglitazone;
- Microtubule inhibitors, such as colchicine, dolastatin 15, nocodazole, docetaxel, paclitaxel, podophyllotoxin, rhizoxin, vinblastine, vincristine, vindesine, vinorelbine (navelbine), vinflunine ;
- Radioisotopes;
- Antisens nucleic aids or siRNAs,
- Other antitumor agents selected from the group consisting of disulfiram, 17-(allylamino)-17-demethoxygeldanamycin, 4-amino-1,8-naphthalimide, apigenin, brefeldin A, cimetidine, dichloromethylene-diphosphonic acid, leuprolide (leuprorelin), luteinizing hormone-releasing hormone, pifithrin-a, rapamycin, thapsigargin, urinary trypsin inhibitor fragment (bikunin).

5. The copolymer according to anyone of claims 1-4, which is a statistic copolymer.

6. The copolymer according to anyone of claims 1-4, which is a block copolymer.

7. A core-shell nanoparticle comprising a core on which is adsorbed by electrostatic, covalent, hydrophobic or hydrogen binding a shell made of the copolymer according to anyone of claims 1-6, wherein said nanoparticle largest dimension is in the range of 1-1000 nm, preferably 10-150 nm.

8. A core-shell nanoparticle, comprising :
a) a core; and
b) a shell comprising:
(b3) an internal shell consisting of an internal polymeric layer linked to the core by electrostatic binding and optionally one or more further internal polymeric layers, wherein each optional successive internal polymeric layer is linked to the preceding internal polymeric layer by electrostatic binding, and the last internal polymeric layer comprises amine groups,
(b4) an external shell consisting of 1+2n external polymeric layers, wherein
- n may be 0 or a positive integer,
- each 1+2i polymeric layer, 0 ≤ i ≤ n, is made of a copolymer according to anyone of claims 1-6, and
- when n ≥ 1, each 2i polymeric layer, 1 ≤ i ≤ n, is a polyamine and is covalently linked to the coupling molecule of the preceding 2i-1 polymeric layer.
wherein said nanoparticle largest dimension is in the range of 1-1000 nm, preferably 10-150 nm.

9. The core-shell nanoparticle according to claim 7 or 8, wherein said core particle is made of a material selected from metals, in particular gold, silver or platinum; magnetic materials; semiconductors, in particular CdSe, ZnS, CdSe/ZnS, ZnSe or SiO₂; metal oxides; colloidal silica; silanized colloidal silica, plastic, in particular polystyrene latex and melamine formaldehyde latexes; biological compounds, in particular proteins, antibodies, DNA, RNA; (polyelectrolyte/polyelectrolyte) complexes; (polyelectrolyte/multivalent ion) complexes, (polyelectrolyte/multivalent ion/metallic nanoparticles) complexes.

10. The core-shell nanoparticle according to claim 8 or 9, comprising only one external layer (n=0).

11. A method for preparing a core-shell particle according to claim 8, comprising:
a) providing a core,
b) depositing onto said core an internal polymeric layer and optionally one or more further internal polymeric layers using electrostatic Layer by Layer (LBL) technology to obtain an internal shell, wherein the last internal polymeric layer comprises monomers with amine groups, and advantageously wherein drugs, proteins, peptides, radionuclide, contrast agents are either chemically coupled or absorbed to the internal polymeric layer, and
c) depositing onto said last internal shell a first external polymeric layer using covalent Layer by Layer (LBL) technology, wherein said first external polymeric layer is made of the copolymer according to anyone of claims 1-6,
d) optionally repeating n times (n≥1) the following steps:
i) depositing onto the preceding external polymeric layer made of the copolymer according to anyone of claims 1-6 a polyamine layer using covalent Layer by Layer (LBL) technology, by reacting the coupling molecules of the copolymer according to anyone of claims 1-6 with the amine groups of said polyamine to form covalent bonds, and
ii) depositing onto the preceding polyamine layer an external polymeric layer made of the copolymer according to anyone of claims 1-6 using covalent Layer by Layer (LBL) technology, by reacting the amine groups of the preceding polyamine layer with the coupling molecules of the copolymer according to anyone of claims 1-6 to form covalent bonds,
wherein the size of the core and the number of internal and external polymer layers are selected in order to obtain a nanoparticle largest dimension in the range of 1-1000 nm, preferably 10-150 nm.

12. A hollow shell nanoparticle consisting of a shell as defined in claims 8 or 10.

13. The hollow shell nanoparticle according to claim 13, wherein the space left by the dissolved core may be filled with bioactive compounds, including drugs, preferably anticancerous drugs; ligands for active sites of proteins; proteins; peptides; polymers; polyelectrolytes; DNA/RNA; fluorophores; quantum dots; or radioisotopes.

14. A method for preparing a hollow shell nanoparticle according to claim 13, comprising:
a) providing a core-shell nanoparticle according to anyone of claims 8-10, and
b) dissolving the core.

15. An un-aggregated composition, comprising a plurality of un-aggregated core-shell nanoparticles according to anyone of claims 7-10 or of un-aggregated hollow shell nanoparticles according to claim 12.

16. A pharmaceutical composition, comprising a core-shell nanoparticle according to anyone of claims 7-10, a hollow shell nanoparticle according to claim 12, or an un-aggregated composition according to claim 14, and a pharmaceutically acceptable carrier.

17. A core-shell nanoparticle according to anyone of claims 7-10, a hollow shell nanoparticle according to claim 12, an un-aggregated composition according to claim 14, or a pharmaceutical composition according to claim 15, for use as a medicament, preferably intended for treating cancer, still more preferably as a tumor-killing heat inducer in combination with an alternative magnetic and/or electromagnetic field in a magnetic and/or electromagnetic hyperthermia treatment.


SEQUENCE LISTING

<110>  Centre National de la Recherche Scientifique (CNRS)
       Université de Strasbourg
       Institute of Macromolecular Chemistry

<120>  Multifunctional Stealth Nanoparticles for Biomedical Use

<130>  D24462

<160>  12

<170>  PatentIn version 3.3

<210>  1
<211>  4
<212>  PRT
<213>  artificial

<220>
<223>  Enzyme-specific cleavable spacer molecule or pH-specific
       cleavable spacer molecule

<400>  1

Leu Ala Leu Ala
1


<210>  2
<211>  4
<212>  PRT
<213>  artificial

<220>
<223>  Enzyme-specific cleavable spacer molecule or pH-specific
       cleavable spacer molecule

<400>  2

Gly Phe Leu Gly
1


<210>  3
<211>  4
<212>  PRT
<213>  artificial

<220>
<223>  Enzyme-specific cleavable spacer molecule or pH-specific
       cleavable spacer molecule

<400>  3

Gly Leu Phe Gly
1


<210>  4
<211>  4

<212>  PRT
<213>  artificial

<220>
<223>  Enzyme-specific cleavable spacer molecule or pH-specific
       cleavable spacer molecule

<400>  4

Gly Phe Phe Gly
1


<210>  5
<211>  4
<212>  PRT
<213>  artificial

<220>
<223>  Enzyme-specific cleavable spacer molecule or pH-specific
       cleavable spacer molecule

<400>  5

Gly Phe Tyr Ala
1


<210>  6
<211>  4
<212>  PRT
<213>  artificial

<220>
<223>  Enzyme-specific cleavable spacer molecule or pH-specific
       cleavable spacer molecule

<400>  6

Gly Phe Ala Leu
1


<210>  7
<211>  5
<212>  PRT
<213>  artificial

<220>
<223>  Enzyme-specific cleavable spacer molecule or pH-specific
       cleavable spacer molecule

<400>  7

Gly Phe Leu Phe Gly
1               5


<210>  8
<211>  5
<212>  PRT

<213>  artificial

<220>
<223>  Enzyme-specific cleavable spacer molecule or pH-specific
       cleavable spacer molecule

<400>  8

Tyr Gly Gly Phe Leu
1               5


<210>  9
<211>  5
<212>  PRT
<213>  artificial

<220>
<223>  Tumor-targeting peptide CREKA

<400>  9

Cys Arg Glu Lys Ala
1               5


<210>  10
<211>  13
<212>  PRT
<213>  artificial

<220>
<223>  Tumor-targeting peptide CTVALPGGYV RVC

<400>  10

Cys Thr Val Ala Leu Pro Gly Gly Tyr Val Arg Val Cys
1               5                   10


<210>  11
<211>  14
<212>  PRT
<213>  artificial

<220>
<223>  Bomberin peptides

<400>  11

Glu Gln Arg Leu Gly Asn Gln Trp Ala Val Gly His Leu Met
1               5                   10


<210>  12
<211>  7
<212>  PRT
<213>  artificial

<220>
<223>  Bomberin peptides

<400>  12

Gln Trp Ala Val Gly His Leu
1               5